# FASCICULE DE BREVET EUROPEEN

(11) **EP 0 995 743 B1**
(45) Date de publication et mention de la délivrance du brevet: **02.04.2003**
(21) Numéro de dépôt: 99402624.3
(22) Date de dépôt: 22.10.1999
(51) Int. Cl.: C07D 221/20, A61K 31/47, C07D 215/20, C07D 471/10, C07D 401/06

(54) **Dérivés de dihydro- et tétrahydroquinoléine en tant qu'antioxydant médical**
Dihydro- und Tetrahydrochinolinderivate als medizinisches Antioxydans
Derivatives of dihydro- and tetrahydrochinoline as a medicinal antioxydans

(30) Priorité: 23.10.1998 FR 9813306
(43) Date de publication de la demande: 26.04.2000
(73) Titulaire: LES LABORATOIRES SERVIER, 92200 Neuilly sur Seine (FR)
(72) Inventeur: Casara, Patrick, 78670 Villennes sur Seine (FR); Dorey, Gilbert, 78000 Versailles (FR); Lestage, Pierre, 78170 la Celle Saint Cloud (FR); Lockhart, Brian, 78290 Croissy sur Seine (FR)

(56) Documents cités:
- EP-A- 0 350 304
- US-A- 4 305 932
- CHEMICAL ABSTRACTS, vol. 128, no. 5, 2 février 1998 (1998-02-02) Columbus, Ohio, US; abstract no. 48145q, SUZUKI, TOMOO ET AL.: "Preparation of malonic acid diamides as antiarteriosclerotics." XP002107502 & JP 09 301953 A (SANWA KAGAKU KENKYUSHO CO., LTD.) -& DATABASE CHEMICAL ABSTRACTS [Online] 128:48145, XP002107505
- CHEMICAL ABSTRACTS, vol. 113, no. 5, 30 juillet 1990 (1990-07-30) Columbus, Ohio, US; abstract no. 40418a, DE, DIBYENDU ET AL.: "Novel synthesis of 6,7-dialkoxy-2,2-dialkyl-3-hydroxyethyl-1, 2,3,4-tetrahydroquinolines." XP002107503 -& DATABASE CHEMICAL ABSTRACTS [Online] CA 113:40418, XP002107506 & INDIAN J. CHEM., SECT. B, vol. 29B(1), - 1990 pages 70-71,
- VASILLA PARTALI ET AL.: "Photo-Emde Degradation of 1,2,3,4-Tetrahydroquinolinium Salts" HELVETICA CHIMICA ACTA., vol. 68, - 1985 pages 1952-1960, XP002107501 BASEL CH
- CHEMICAL ABSTRACTS, vol. 92, no. 18, 5 mai 1980 (1980-05-05) Columbus, Ohio, US; abstract no. 149667e, SHMULOVICH, V. G. ET AL.: "Study of the antioxidant activity of inhibitors of the oxidation of paraffin oil hydrocarbons." XP002107504 & NEFTEKHIMIYA, vol. 19, no. 6, - 1979 pages 912-920,

## Description

La présente invention concerne de nouveaux composés dihydro et tétrahydroquinoléiniques, leur procédé de préparation et les compositions pharmaceutiques qui les contiennent.

La préparation de dérivés de structure 1,2-dihydroquinoléine a été décrite dans les brevets DD 227434 et DE 4115535. D'autres composés possédant ce même noyau diversement substitué ont été utilisés dans la préparation de viandes fumées (GB 1537334) ou comme marqueurs photochimiques (WO 8905994). Les propriétés antioxydantes de dérivés dihydro et tétrahydroquinoléiniques ont été utilisées dans le domaine des lubrifiants et huiles (EP 072349 ; V.G. SHMULOVICH et al., Neftekhimiya, 1979, 19 ; EP 350304). Ce type de composés a également été décrit en tant qu'inhibiteurs de l'absorption lipidique (EP 028765) ou encore comme agents de traitements des déficiences en vitamines E (US 4305932).

Selon la théorie radicalaire du vieillissement de Hartman (J. Gérontol., 1956, 11, 298) les agressions oxydantes successives créent des conditions de stress oxydant qui traduisent un déséquilibre dans l'organisme entre les systèmes producteurs d'espèces radicalaires et les systèmes protecteurs vis à vis de ces espèces (R.E. PACIFICI, K.J.A. DAVIES, Gerontology, 1991, 37, 166). Différents mécanismes de défense peuvent agir en synergie et permettent de contrôler l'action des radicaux libres. Ces mécanismes peuvent être enzymatiques, comme c'est le cas pour des systèmes faisant intervenir la superoxyde dismutase, la catalase, et la glutathion peroxydase, ou non enzymatique dans le cas de l'intervention des vitamines E et C. Toutefois avec l'âge ces protections naturelles deviennent de moins en moins efficaces, en particulier à cause de l'inactivation oxydative de nombreuses enzymes (A. CASTRES de PAULET, Ann. Biol. Clin., 1990, 48, 323).

Les conditions de stress oxydant ont pu être associées à des affections liées à la sénescence que sont l'athérosclérose, la cataracte, le diabète non insulino-dépendant ou le cancer (M. HAYN et al., Life Science, 1996, 59, 537). Le système nerveux central est particulièrement sensible au stress oxydant en raison de sa haute consommation d'oxygène, du niveau relativement faible de ses défenses antioxydantes, et de la forte teneur en fer de certaines aires cérébrales (S.A. BENKOVIC et al., J. Comp. Neurol. 1993, 338, 92 ; D. HARTMAN, Drugs Aging, 1993, 3, 60). Les agressions oxydantes successives constituent donc un des facteurs étiologiques principaux du vieillissement cérébral et des maladies associées que sont la maladie d'Alzheimer, et les maladies neurodégénératives chroniques, les neurodégénérescences des ganglions de la base (maladie de Parkinson, Hungtington,...), (B. HALLIWELL, J. Neurochem., 1992, 59, 1609).

Les composés de la présente invention, outre le fait qu'ils soient nouveaux présentent des propriétés pharmacologiques intéressantes. Leur caractère antioxydant, piégeur d'espèces oxygénées réactives, en particulier au niveau du système nerveux central, permet d'envisager leur utilisation pour s'opposer aux effets des stress oxydants, en particulier au niveau cérébral. La plupart d'entre eux présentent de plus l'avantage de ne pas provoquer d'effet hypothermisant aux doses utilisées pour obtenir une action neuroprotectrice. Ils seront donc utiles dans le traitement d'affections liées à la sénescence, comme l'athérosclérose ou la cataracte, dans le traitement du cancer, dans le traitement des troubles cognitifs, et dans le traitement des maladies neurodégénératives aiguës telles que l'ischémie cérébrale et l'épilepsie et dans le traitement des maladies neurodégénératives chroniques telles que la maladie d'Alzheimer, la maladie de Pick ou les neurodégénérescences des ganglions de la base (maladie de Parkinson, Hungtington).

La présente invention concerne particulièrement les composés de formule générale (I): 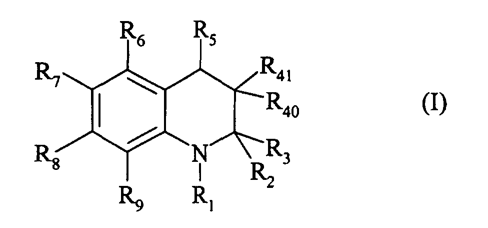 dans laquelle :
- R₁ représente un atome d'hydrogène,
- R₂ et R₃ représentent chacun un groupement alkyle,
   ou bien,
   R₂ et R₃ forment ensemble avec l'atome de carbone qui les porte un groupement cycloalkyle (C₃-C₈) ou un groupement hétérocycloalkyle monocyclique non substitué
   ou substitué par un groupement alkyle, cycloalkyle, cycloalkylalkyle, aryle ou arylalkyle,
- R₄₀ représente un atome d'hydrogène ou un groupement -V-Q dans lequel V représente un groupement alkylène et Q représente un groupement hétérocycloalkyle,
- R₄₁ et R₅ forment ensemble une liaison, ou représentent chacun un atome d'hydrogène,
- R₆, R₇, R₈ et R₉ représentent indépendamment un atome d'hydrogène, un groupement alkyle ou un groupement -OW dans lequel W représente un groupement alkyle, acyle, ou phényle (sachant que R₆, R₇, R₈ et R₉ ne peuvent tous représenter simultanément un atome d'hydrogène et qu'au moins un d'entre eux représente un groupement -OW tel que défini précédemment),
   à la condition que :
   - lorsque R₂ et R₃ représentent un groupement alkyle :
      - si R₆ à R₉ représentent indépendamment un atome d'hydrogène, un groupement alkyle, ou un groupement -OW dans lequel W représente un groupement alkyle, tandis que R₄₁ et R₅ forment ensemble une liaison, alors R₄₀ est différent d'un atome d'hydrogène,
      - le composé de formule (I) étant différent de la 6-méthoxy-2,2-diméthyl-1,2,3,4-tétrahydroquinoline,
      - le composé de formule (I) étant différent de la 6,8-diméthoxy-2,2-diméthyl-1,2,3,4-tétrahydroquinoline,
   étant entendu que :
   - le terme alkyle désigne une chaîne de 1 à 6 atomes de carbone, linéaire ou ramifiée,
   - le terme acyle désigne un groupement alkyle-carbonyle, alkyle ayant la même signification que précédemment,
   - le terme alkényle désigne une chaîne de 2 à 6 atomes de carbone, linéaire ou ramifiée, contenant de 1 à 3 double(s) liaison(s),
   - le terme alkynyle désigne une chaîne de 2 à 6 atomes de carbone, linéaire ou ramifiée, contenant de 1 à 3 triple(s) liaison(s),
   - le terme alkylène désigne un groupement bivalent linéaire ou ramifié contenant de 1 à 6 atomes de carbone,
   - le terme alkénylène désigne un groupement bivalent linéaire ou ramifié contenant de 2 à 6 atomes de carbone et de 1 à 3 doubles liaisons,
   - le terme alkynylène désigne un groupement bivalent linéaire ou ramifié contenant de 2 à 6 atomes de carbone et de 1 à 3 triples liaisons,
   - le terme aryle représente un groupement phényle, naphtyle ou biphényle,
   - le terme hétérocycloalkyle désigne un groupement mono ou bicyclique de 4 à 11 chaînons contenant de 1 à 6 hétéroatomes choisis parmi azote, oxygène et soufre, ce groupement pouvant comporter une ou plusieurs insaturations sans pour autant présenter un caractère aromatique,
   - le terme substitué affecté aux expressions aryle, et arylalkyle, signifie que les groupements concernés sont substitués par un ou plusieurs atomes d'halogène, ou groupements alkyle, alkoxy (C₁-C₆) linéaire ou ramifié, perhalogénoalkyle (C₁-C₆) linéaire ou ramifié, amino (éventuellement substitué par 1 ou 2 groupements alkyle), cyano, carboxy, alkoxycarbonyl (C₁-C₆) linéaire ou ramifié, aminocarbonyle (éventuellement substitué sur l'atome d'azote par 1 ou 2 groupements alkyle), nitro, ou hydroxy,
   - le terme substitué affecté aux expressions alkyle, alkényle, alkynyle, et cycloalkyle, signifie que ces groupements sont substitués par un ou plusieurs groupements choisis parmi hydroxy, alkoxy (C₁-C₆) linéaire ou ramifié, alkylthio (C₁-C₆) linéaire ou ramifié, amino (éventuellement substitué par un ou deux groupements alkyle), carboxy, nitro, cyano, alkoxycarbonyl (C₁-C₆) linéaire ou ramifié, aminocarbonyle (éventuellement substitué sur l'atome d'azote par un ou deux groupements alkyle),
   - le terme substitué affecté aux expressions hétérocycloalkyle, signifie que les groupements concernés sont substitués par un ou plusieurs atomes d'halogène, ou groupements alkyle, alkoxy (C₁-C₆) linéaire ou ramifié, perhalogénoalkyle (C₁-C₆) linéaire ou ramifié, amino (éventuellement substitué par 1 ou 2 groupements alkyle), cyano, carboxy, alkoxycarbonyl (C₁-C₆) linéaire ou ramifié, aminocarbonyle (éventuellement substitué sur l'atome d'azote par 1 ou 2 groupements alkyle), nitro, hydroxy, ou oxo,
leurs énantiomères, diastéréoisomères, ainsi que leurs sels d'addition à un acide ou à une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthanesulfonique, camphorique, oxalique, etc...

Parmi les bases pharmaceutiquement acceptables, on peut citer à titre non limitatif l'hydroxyde de sodium, l'hydroxyde de potassium, la triéthylamine, la tertbutylamine, etc...

De façon préférentielle, dans les composés de formule (I), R₆, R₇, R₈ et R₉ représentent indépendamment un atome d'hydrogène, un groupement alkyle, ou un groupement -OW dans lequel W représente un groupement alkyle, acyle ou phényle. Plus particulièrement W représente un groupement alkyle.

Des composés préférés de l'invention sont ceux pour lesquels R₂ et R₃ représentent chacun un groupement alkyle, par exemple méthyle.

D'autres composés préférés de l'invention sont ceux pour lesquels R₂ et R₃ forment ensemble un groupement cycloalkyle ou hétérocycloalkyle éventuellement substitué, et plus particulièrement un groupement cycloalkyle, par exemple cyclohexyle.

Dans les composés de formule (I) R₄₀ représente préférentiellement un atome d'hydrogène ou un groupement V-Q, V étant plus particulièrement un groupement alkylène, et Q plus particulièrement un groupement hétérocycloalkyle.

Le groupement aryle préféré de l'invention est le groupement phényle.

Un aspect avantageux de l'invention concerne les composés de formule (I) pour lesquels R₁ représente un atome d'hydrogène, R₂ et R₃ représentent un groupement alkyle ou forment ensemble un groupement cycloalkyle, R₄₀ représente un atome d'hydrogène ou un groupement -V-Q dans lequel V représente un groupement alkylène et Q représente un groupement hétérocycloalkyle, et R₆, R₇, R₈, R₉ représentent indépendamment un atome d'hydrogène, un groupement alkyle, ou un groupement -OW dans lequel W représente un groupement alkyle, acyle ou phényle, sachant que R₆, R₇, R₈ et R₉ ne peuvent tous représenter un atome d'hydrogène et qu'au moins un d'entre eux représente un groupement -OW tel que défini précédemment.

Parmi les composés préférés de l'invention, on peut citer :
- 6-Ethoxy-1,2-dihydroquinoléine-2-spirocyclohexane,
- 6-Ethoxy-5,7,8-triméthyl-1,2-dihydroquinoléine-2-spirocyclohexane,
- 8-Ethoxy-1,2-dihydroquinoléine-2-spirocyclohexane,
- 5,7-Diisopropyl-6-éthoxy-1,2-dihydroquinoléine-2-spirocyclohexane,
- 5,7-Diméthyl-6-éthoxy-1,2-dihydroquinoléine-2-spirocyclohexane,
- 6-Ethoxy-2,2,5,7,8-pentaméthyl-1,2,3,4-tétrahydroquinoléine,
- 5,7-Diisopropyl-2,2-diméthyl-6-éthoxy-1,2,3,4-tétrahydro-quinoléine,
- 6-Ethoxy-2,2,5,7-tétramethyl-1,2,3,4-tétrahydroquinoléine,
- 6-Ethoxy-5,7,8-triméthyl-1,2,3,4-tétrahydroquinoléine-2-spirocyclohexane,
- 6-Ethoxy-1,2-dihydroquinoléine-2-spiro-4'-pipéridine,
- 6-Ethoxy-1,2-dihydroquinoléine-2-spiro-4'-(1'-cyclopropylméthyl-pipéridine)
- 2,2-Diméthyl-6-éthoxy-3-(2-morpholinoéthyl)-1,2-dihydroquinoléine, dichlorhydrate
- 6-tert-butoxy-1,2-dihydroquinoléine-2-spirocyclohexane,
- 6-méthoxy-1,2-dihydroquinoléine-2-spirocyclohexane,
- 6-phénoxy-1,2-dihydroquinoléine-2-spirocyclohexane,
- 6-Ethoxy-5,7-diméthyl-1,2,3,4-tétrahydroquinoléine-2-spirocyclohexane,
- 6-Ethoxy-2,2-diméthyl-3[2-(2,6-dioxopipérazin-4-yl)éthyl]-1,2-dihydroquinoléine,
- 6-Ethoxy-2,2-diméthyl-3[2-(1-pipéridinyl)éthyl]-1,2-dihydroquinoléine,
- 2[2-(6-Ethoxy-2,2-diméthyl-1,2-dihydro-3-yl)éthyl]-1H-isoindole-1,3(2H)-dione,
- 3[2-(6-Ethoxy-2,2-diméthyl-1,2-dihydro-3-quinolényl)éthyl]-4(3H)-quinazolinone,
- 6-tert-butylcarbonyloxy-1,2-dihydroquinoléine-2-spirocyclohexane,
et tout particulièrement les :
- 6-Ethoxy-2,2,5,7,8-pentaméthyl-1,2,3,4-tétrahydroquinoléine,
- 6-Ethoxy-1,2-dihydroquinoléine-2-spirocyclohexane.

La présente invention concerne également le procédé de préparation des composés de formule (I), caractérisé en ce que l'on utilise comme produit de départ une aniline diversement substituée de formule (II) : 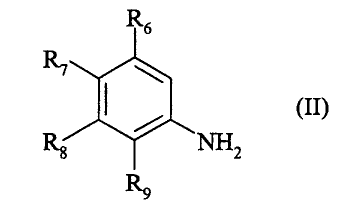 dans laquelle R₆, R₇, R₈, R₉, sont tels que définis dans la formule (I),
qui est soumis :
- soit à l'action, en milieu basique et éventuellement en présence d'un catalyseur, d'un acétylénure halogéné de formule (III) : 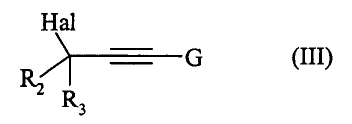 dans laquelle R₂ et R₃ sont tels que définis dans la formule (I), G représente un atome d'hydrogène ou un groupement trialkylsilyle, et Hal représente un atome d'halogène,
   pour conduire à un composé de formule (IV) : 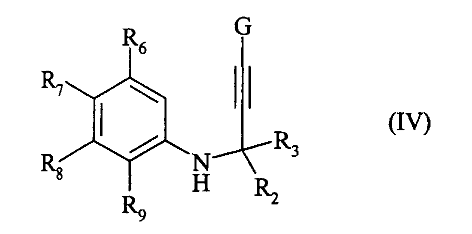 dans laquelle R₂, R₃, R₆, R₇, R₈, R₉ et G sont tels que définis précédemment,
- soit à l'action d'un dérivé carbonylé de formule (V) : 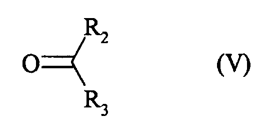 dans laquelle R₂ et R₃ sont tels que définis dans la formule (I),
   pour conduire à un composé de formule (VI) : 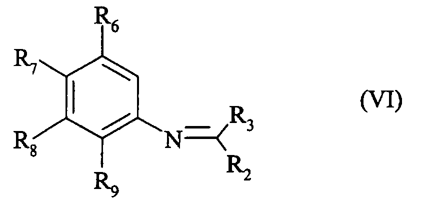 dans laquelle R₂, R₃, R₆, R₇, R₈ et R₉ sont tels que définis précédemment,
   composé (VI) qui est soumis, en milieu basique, à l'action d'un acétylénure de formule (III) telle que définie précédemment,
   pour conduire à un composé de formule (IV) telle que précédemment définie,
   composé de formule (IV), qui après coupure éventuelle du groupement trialkylsilyle, est cyclisé par chauffage en présence d'un catalyseur approprié, pour conduire à un composé de formule (I/a) : 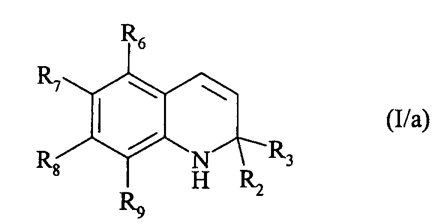 cas particulier des composés de formule (I) pour lequel R₂, R₃, R₆, R₇, R₈ et R₉, sont tels que définis précédemment,
qui peut être :
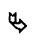 soit soumis à une réaction de réduction pour conduire à un composé de formule (I/b) : 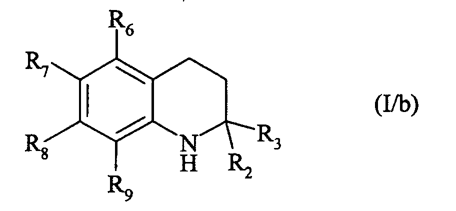 cas particulier des composés de formule (I) pour lequel R₂, R₃, R₆, R₇, R₈ et R₉, sont tels que définis précédemment,
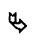 soit, après protection de l'atome d'azote cyclique, soumis successivement à une réaction d'hydroxyhalogénation et à une réaction d'oxydation en position benzylique, pour conduire à un composé de formule (VIII) : 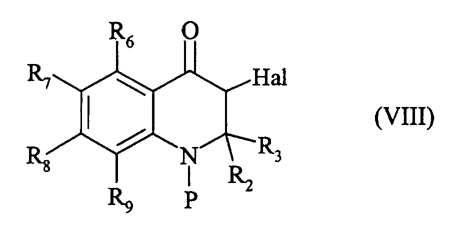 dans laquelle R₂, R₃, R₆, R₇, R₈, R₉ sont tels que définis précédemment, Hal représente un atome d'halogène, et P est un groupement protecteur de l'azote cyclique (par exemple un groupement acétyle, trifluoroacétyle, tertbutoxycarbonyle ou benzyloxycarbonyle),
qui est soumis à une réaction de substitution nucléophile pour conduire à un composé de formule (IX) : 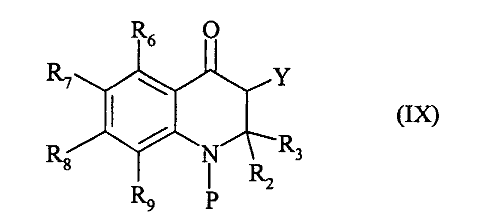 dans laquelle R₂, R₃, R₆, R₇, R₈, R₉ et P sont tels que définis précédemment, et Y représente soit un groupement R₄₀ différent d'un atome d'hydrogène tel que défini dans la formule (I), soit un précurseur de ce groupement,
qui, après déprotection de l'azote cyclique, subit une réaction de réduction de la fonction carbonyle, suivie d'une élimination, pour conduire à un composé de formule (X) : 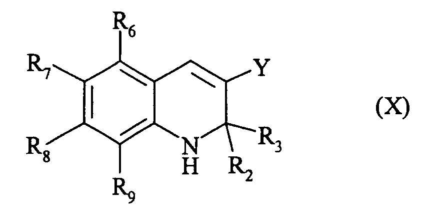 dans laquelle R₂, R₃, R₆, R₇, R₈, R₉ et Y sont tels que définis précédemment,
composé de formule (X) qui peut, lorsque Y est un précurseur d'un groupement R₄₀ tel que défini précédemment, subir une suite de réactions classiques dans le but de conduire à un composé de formule (I/c) : 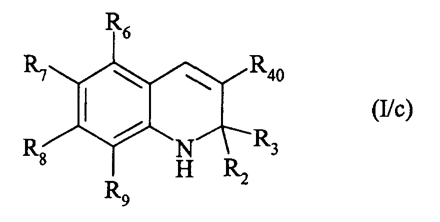 cas particulier des composés de formule (I) pour lequel R₂, R₃, R₆, R₇, R₈, R₉ et R₄₀ sont tels que définis précédemment,
qui peut être réduit pour conduire à un composé de formule (I/d) : 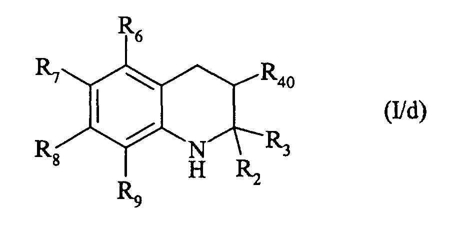 cas particulier des composés de formule (I) pour lequel R₂, R₃, R₄₀, R₆, R₇, R₈ et R₉ sont tels que définis précédemment,
composés (I/a) à (I/d), formant la totalité des composés de formule (I) et :
- qui peuvent être, le cas échéant, purifiés selon une technique classique de purification,
- dont on sépare, le cas échéant, les stéréoisomères selon une technique classique de séparation,
- que l'on transforme, si on le souhaite, en leurs sels d'addition à un acide ou à une base pharmaceutiquement acceptable.

La présente invention a également pour objet les compositions pharmaceutiques renfermant comme principe actif au moins un composé de formule (I) seul ou en combinaison avec un ou plusieurs excipients ou véhicules inertes non toxiques.

Parmi les compositions pharmaceutiques selon l'invention, on pourra citer, plus particulièrement celles qui conviennent pour l'administration orale, parentérale, nasale, les comprimés simples ou dragéifiés, les comprimés sublinguaux, les gélules, les tablettes, les suppositoires, les crèmes, les pommades, les gels dermiques, etc...

La posologie varie selon le sexe, l'âge et le poids du patient, la nature et la sévérité de l'affection ainsi que la voie d'administration. Celle-ci peut être orale, nasale ou parentérale. D'une manière générale, la posologie unitaire s'échelonne entre 0,1 et 500 mg pour un traitement en 1 à 3 prises par 24 heures.

Les exemples suivants illustrent l'invention, mais ne la limitent en aucune façon.

Les produits de départ utilisés sont des produits connus ou préparés selon des modes opératoires connus.

### Préparation A : 4-Ethoxy-3,5,6-triméthylaniline

### Stade 1 : 2-Ethoxy-1,3,4-triméthylbenzène

A une solution de 367 mmol (50 g) de 2,3,6-triméthylphénol dans 1500 ml d'acétonitrile sont ajoutées successivement 734 mmol (101,5 g) de carbonate de potassium et 917 mmol (143,1 g) d'iodure d'éthyle. L'ensemble est chauffé à reflux pendant 48 h. Le mélange réactionnel est alors refroidi puis filtré et le filtrat concentré. Le résidu obtenu est mis en solution dans l'acétate d'éthyle et lavé à l'eau puis par une solution aqueuse de chlorure de sodium à 10 %. Après séchage de la phase organique puis concentration, on obtient un résidu huileux qui est purifié par chromatographie sur gel de silice en utilisant comme éluant un mélange éther de pétrole : acétate d'éthyle; 95 : 5, pour conduire au produit attendu.

### Stade 2 : 4-Ethoxy-3,5,6-triméthyl-iodobenzène

A une solution de 171 mmol (28,03 g) de composé décrit au stade précédent, dans 650 ml d'acétonitrile sont ajoutées 222 mmol (50g) de N-iodosuccinimide et l'ensemble est chauffé à reflux pendant 24 h. Le solvant est alors évaporé sous vide et le résidu est repris dans l'éther. La solution est lavée avec une solution saturée en NaHCO₃ puis par une solution aqueuse de chlorure de sodium à 10 %. La phase organique est séchée et concentrée. Le résidu huileux obtenu est purifié par chromatographie sur gel de silice en utilisant comme éluant un mélange éther de pétrole : acétate d'éthyle; 95 : 5, pour conduire au produit attendu.

### Stade 3 : 4-Ethoxy-3,5,6-triméthylaniline

Dans un ballon de 1 l sont placées, sous atmosphère inerte, 0,46 mmol (0,426 g) de Pd₂(dba)₃ et 1,39 mmol (0,947 g) de BINAP. Dans un second ballon sont mises en solution dans 250 ml de THF anhydre, 46,5 mmol (13,5 g) du composé décrit au stade précédent, 65,1 mmol (6,26g) de tert-butylate de sodium, 65,1 mmol (17,21 g) de 18-C-6 et 65,8 mmol (10,12 g) de benzophenone imine. La solution du second ballon est introduite à l'aide d'une canne de transfert dans le ballon contenant le système catalytique. L'ensemble est chauffé à 60°C pendant 3 h puis le mélange réactionnel est dilué à l'éther. Le précipité formé est filtré sur verre fritté puis le filtrat évaporé. Le résidu obtenu est remis en solution dans 300 ml de THF. On y ajoute 30 ml d'une solution d'acide chlorhydrique (2N) et on place la solution sous agitation à température ambiante pendant 1 h. L'ensemble est alors dilué dans un excès d'acide chlorhydrique (1N) et un mélange heptane : acétate d'éthyle ; 2 : 1. La phase aqueuse est séparée puis neutralisée à l'aide d'une solution 1M de soude. Après extraction au dichlorométhane, séchage de la phase organique et évaporation du solvant, le produit attendu est obtenu.

### Préparation B : 3,5-Diisopropyl-4-éthoxyaniline

### Stade 1 : 2,6-Diisopropyl-4-nitrophénol

A une solution de 53,9 mmol (9,62 g) de 2,6-diisopropylphénol dans 350 ml d'acide acétique refroidie à 0°C sont additionnées goutte-à-goutte 78,3 mmol (4,93 g) d'acide nitrique fumant. Le mélange réactionnel est placé sous agitation à 0°C pendant 1 h 30 min puis celui-ci est versé dans un mélange d'acétate d'éthyle et de glace. La phase organique est isolée puis lavée à l'eau. Après séchage et évaporation du solvant on récupère un résidu huileux que l'on purifie par chromatographie sur gel de silice en utilisant comme éluant un mélange éther de pétrole : acétate d'éthyle ; 9 : 1.

### Stade 2 : 2,6-Diisopropyl-1-éthoxy-4-nitrobenzène

Le produit attendu et obtenu selon le procédé décrit au stade 1 de la préparation A, à partir du composé décrit au stade précédent.

### Stade 3 : 3,5-Diisopropyl-4-éthoxyaniline

Une solution de 19,9 mmol (5,0 g) du composé décrit au stade précédent dans 135 ml d'éthanol absolu en présence de 1,5 g de Palladium sur charbon (10 %) est placée sous 1 atm d'hydrogène à température ambiante pendant 4 heures. Après cette période, le mélange réactionnel est filtré puis le filtrat concentré pour conduire au composé attendu.

### Préparation C : 3,5-Diméthyl-4-éthoxyaniline

### Stade 1 : 3,5-Diméthyl-4-éthoxy-nitrobenzène

A une solution de 149,5 mmol (25 g) de 2,6-diméthyl-4-nitrophénol dans 1300 ml d'acétonitrile sont ajoutées successivement 300 mmol (326 g) de carbonate de césium et 374 mmol (58,5 g) d'iodure d'éthyle. L'ensemble est chauffé à reflux sous atmosphère inerte pendant 15 h. Le mélange réactionnel est alors refroidi puis filtré et le filtrat évaporé. Le résidu est mis en solution dans l'acétate d'éthyle et lavé à l'eau puis par une solution aqueuse de chlorure de sodium à 10%. Après séchage de la phase organique puis concentration, on obtient le produit attendu.

### Stade 2 : 3,5-Diméthyl-4-éthoxyaniline

Une solution de 149,5 mmol (29,19 g) du composé décrit au stade précédent dans 1000 ml d'éthanol absolu en présence de 9,4 g de Palladium sur charbon (10 %) est placée sous 1 atm d'hydrogène à température ambiante pendant 4 heures. Après cette période, le mélange réactionnel est filtré puis le filtrat concentré pour conduire au composé attendu.

### Préparation D : 4-tert-butoxy-aniline

### Stade 1 : 1-tert-butoxy-4 -nitrobenzène

A une solution de 10,78 mmol (1,5 g) de 4-nitrophénol dans 10ml de toluène sont additionnées, à température ambiante, 43,79 mmol de N-N-diméthylformamide di-tert-butylacétal. Le milieu réactionnel est chauffé à reflux sous forte agitation pendant 5 h. Le mélange réactionnel est dilué à l'acétate d'éthyle puis lavé à l'eau, par une solution aqueuse saturée de bicarbonate de sodium puis à l'aide d'une solution aqueuse de chlorure de sodium à 10 %. Après séchage de la phase organique puis concentration on obtient le produit attendu.

### Stade 2 : 4-tert-butoxy aniline

Une solution de 4,61 mmol (0,90 g) du composé décrit au stade précédent dans 15 ml d'éthanol absolu contenant 24,65 mmol (2,12 g) de cyclohexene et 0,29 g de Palladium sur charbon (10 %) est chauffée à reflux sous forte agitation pendant 2 h. Après cette période, le mélange réactionnel est filtré puis le filtrat concentré pour conduire au composé attendu.

### Préparation E : 4-tert-butylcarbonyloxy-aniline

### Stade 1 : 1-tert-butylcarbonyloxy-4-nitrobenzène

A une solution de 107,8 mmol (15g) de 4-nitrophénol dans 250 ml de pyridine sont additionnées, à 0°C, 161,1 mmol (19,48 g) de chlorure de pyvaloyle. Le milieu réactionnel est laissé sous agitation pendant 72 h à température ambiante. Le mélange réactionnel est évaporé sous vide et le résidu huileux est dilué à l'acétate d'éthyle puis lavé à l'aide d'une solution aqueuse d'acide chlorhydrique (0,1 N) puis par un solution aqueuse de chlorure de sodium à 10 %. Après séchage de la phase organique puis concentration on obtient le produit attendu.

### Stade 2 : 4-tert-butylcarbonyloxy-aniline

Une solution de 95,41 mmol (21,30 g) du composé décrit au stade précédent, dans 1200 ml d'un mélange 4/1 de méthanol et d'acide acétique contenant 573 mmol (32,00 g) de fer, est chauffée à 65°C pendant 20 h. Après cette période, le mélange réactionnel est filtré puis le filtrat concentré pour donner un résidu huileux que l'on dilue à l'acétate d'éthyle et que l'on lave par une solution aqueuse de bicarbonate de sodium à 10 % puis par une solution aqueuse de chlorure de sodium à 10 %. Après séchage de la phase organique puis concentration on obtient le produit attendu.

### EXEMPLE 1 : 6-Ethoxy-1,2-dihydroquinoléine-2-spirocyclohexane, chlorhydrate

### Etape 1 : N-(4-Ethoxyphényl)-N-[1-(1-éthynyl)cyclohexyl]amine

A une solution de 18,2 mmol (2,5 g) de p-phénétidine dans un mélange éther : eau (4:1; 30 ml) contenant 24,5 mmol (3,42 ml) de triéthylamine, refroidie à 10°C, sont ajoutées successivement 0,25 mmol (0,025 g) de CuCl, 0,39 mmol (0,025 g) de Cu et goutte-à-goutte 28,0 mmol (4,0 g) de 1-éthynyl-1-chlorocyclohexane préparé suivant la méthode décrite dans J. Am. Chem. Soc., 83, 725, 1961, à partir du 1-éthynylcyclohexanol. Après 4 h d'agitation (en laissant la température évoluer vers la température ambiante) le mélange réactionnel est dilué à l'éther puis lavé avec une solution 1N d'acide sulfurique. La phase éthérée est écartée et la phase aqueuse est neutralisée (à 0°C) à l'aide de pastilles de potasse, puis réextraite à l'éther. La phase organique est séchée puis concentrée pour conduire au produit attendu.

### Etape 2 : 6-Ethoxy-1,2-dihydroquinoléine-2-spirocyclohexane, chlorhydrate

A une solution de 14,79 mmol (2,89 g) du composé décrit à l'étape précédente dans 20 ml de toluène sont ajoutées 3,0 mmol (0,30 g) de CuCl et l'ensemble est chauffé à reflux pendant 40 min. Le solvant est alors évaporé et le résidu huileux est purifié par chromatographie sur gel de silice en utilisant le dichlorométhane comme éluant, pour conduire au produit attendu.
Ce dernier est précipité sous forme de chlorhydrate dans un mélange éther chlorhydrique/isopropanol pour conduire au chlorhydrate correspondant.
*Point de fusion :* 183°- 186°C (décomposition, iPr₂O/CH₂Cl₂).

| *Microanalyse élémentaire* (formule brute : C₁₆ H₂₁ NO.HCl) | | | | |
|---|---|---|---|---|
| | C | H | N | Cl |
| **% trouvé** | 68,50 | 7,87 | 5,08 | 12,67 |
| **% calculé** | 68,68 | 7,92 | 5,01 | 12,61 |

### EXEMPLE 2 : 6-Ethoxy-5,7,8-triméthyl-1,2-dihydroquinoléine-2-spirocyclohexane, chlorhydrate

### Etape 1 : N-(4-Ethoxy-2,3,5-triméthylphényl)-N-[1-(1-éthynyl)cyclohexyl]amine

Le produit attendu est obtenu selon le procédé décrit à l'étape 1 de l'exemple 1, à partir du composé décrit dans la Préparation A.

### Etape 2 : 6-Ethoxy-5,7,8-triméthyl-1,2-dihydroquinoléine-2-spirocyclohexane, chlorhydrate

Le produit attendu est obtenu selon le procédé décrit à l'étape 2 de l'exemple 1, à partir du composé décrit au stade précédent.
Le chlorhydrate correspondant est obtenu par précipitation dans un mélange éther/dichlorométhane auquel on ajoute de l'éther chlorhydrique.
*Point de fusion :* 158°-161°C (Et₂O/CH₂Cl₂)

| *Microanalyse élémentaire* (formule brute : C₁₉ H₂₇ N O.HCl) | | | | |
|---|---|---|---|---|
| | C | H | N | Cl |
| **% trouvé** | 71,13 | 8,68 | 4,30 | 11,24 |
| **% calculé** | 70,90 | 8,77 | 4,35 | 11,01 |

### EXEMPLE 3 : 8-Ethoxy-1,2-dihydroquinoléine-2-spirocyclohexane, chlorhydrate

### Etape 1 : N-(2-Ethoxyphényl)-N-[1-(1-éthynyl)cyclohexyl]amine

Le produit attendu est obtenu selon le procédé décrit à l'étape 1 de l'exemple 1, en remplaçant la p-phénétidine par l'o-phénétidine.

### Etape 2 : 8 -Ethoxy-1,2-dihydroquinoléine-2-spirocyclohexane, chlorhydrate

Le produit attendu est obtenu selon le procédé décrit à l'étape 2 de l'exemple 1, à partir du composé décrit à l'étape précédente. Le chlorhydrate correspondant est obtenu par précipitation dans un mélange éther/dichlorométhane auquel on ajoute de l'éther chlorhydrique.
*Point de fusion :* 154°-155°C (Et₂O/CH₂Cl₂)

| *Microanalyse élémentaire* (formule brute : C₁₆ H₂₁ N O.HCl) | | | | |
|---|---|---|---|---|
| | C | H | N | Cl |
| **% trouvé** | 68,62 | 7,65 | 4,84 | 12,59 |
| **% calculé** | 68,68 | 7,92 | 5,01 | 12,67 |

### EXEMPLE 4 : 5,7-Diisopropyl-6-éthoxy-1,2-dihydroquinoléine-2-spirocyclohexane, chlorhydrate

### Etape 1 : N-(3,5-Dusopropyl-4-éthoxyphényl)-N-[1-(1-éthynyl)cyclohexyl]amine

Le produit attendu est obtenu selon le procédé décrit à l'étape 1 de l'exemple 1, en utilisant comme produit de départ le composé décrit dans la Préparation B.

### Etape 2 : 5,7-Diisopropyl-6-éthoxy-1,2-dihydroquinoléine-2-spirocyclohexane, chlorhydrate

Le produit attendu est obtenu selon le procédé décrit à l'étape 2 de l'exemple 1, à partir du composé décrit au stade précédent.
*Point de fusion :* 166°-169°C (avec décomposition ; Et₂O)

| *Microanalyse élémentaire* (formule brute : C₂₂ H₃₃ N O.HCl | | | | |
|---|---|---|---|---|
| | C | H | N | Cl |
| **% trouvé** | 72,87 | 9,28 | 3,87 | 9,76 |
| **% calculé** | 72,60 | 9,42 | 3,85 | 9,74 |

### EXEMPLE 5 : 5,7-Diméthyl-6-éthoxy-1,2-dihydroquinoléine-2-spirocyclohexane, chlorhydrate

### Etape 1 : N-(3,5-Diméthyl-4-éthoxyphényl)-N[1-(1-éthynyl)cyclohexyl]amine

Le produit attendu est obtenu selon le procédé décrit à l'étape 1 de l'exemple 1, en utilisant comme produit de départ le composé décrit dans la Préparation C.

### Etape 2 : 5,7-Diméthyl-6-éthoxy-1,2-dihydroquinoléine-2-spirocyclohexane, chlorhydrate

Le produit attendu est obtenu selon le procédé décrit à l'étape 2 de l'exemple 1, à partir du composé décrit au stade précédent.
*Point de fusion :* 175°-180°C (Et₂O)

| *Microanalyse élémentaire* (formule brute : C₁₈H₂₅ N O.HCl) | | | | |
|---|---|---|---|---|
| | C | H | N | Cl |
| **% trouvé** | 70,47 | 8,55 | 4,57 | 11,56 |
| **% calculé** | 70,23 | 8,51 | 4,55 | 11,52 |

### EXEMPLE 6 : 6-Ethoxy-2,2,5,7,8-pentaméthyl-1,2,3,4-tétrahydroquinoléine, chlorhydrate

### Etape 1 : N-(1,1-Diméthyl-2-propynyl)-N-(4-éthoxy-2,3,5-triméthylphényl)amine

Le produit attendu est obtenu selon le procédé décrit à l'étape 1 de l'exemple 1, en utilisant comme produit de départ le composé décrit dans la Préparation A, et en remplaçant le 1-éthynyl-1-chlorocyclohexane par le 2-chloro-2-méthyl-3-butyne (préparé suivant la méthode décrite dans J. Am. Chem. Soc., 83, 725, 1961, à partir du 2-méthyl-3-butyn-2-ol).

### Etape 2 : 6-Ethoxy-2,2,5,7,8 pentaméthyl-1,2-dihydroquinoléine

Le produit attendu est obtenu selon le procédé décrit à l'étape 2 de l'exemple 1, à partir du composé décrit au stade précédent.

### Etape 3 : 6-Ethoxy-2,2,5,7,8-pentaméthyl-1,2,3,4-tétrahydroquinoléine, chlorhydrate

A une solution de 5,99 mmol (1,47 g) du composé décrit à l'étape précédente dans 55 ml d'éthanol sont ajoutés 0,9 g de Palladium sur charbon (10 %). La réaction est agitée sous 1 atm d'hydrogène à température ambiante pendant deux heures. Le milieu réactionnel est ensuite filtré, et le filtrat concentré. Le produit attendu est obtenu sous forme de chlorhydrate par précipitation dans l'éther chlorhydrique.

| *Microanalyse élémentaire* (formule brute : C₁₆ H₂₅ N O.HCl) | | | | |
|---|---|---|---|---|
| | C | H | N | Cl |
| **% trouvé** | 67,64 | 9,16 | 5,01 | 12,40 |
| **% calculé** | 67,71 | 9,23 | 4,93 | 12,49 |

### EXEMPLE 7 : 5,7-Diisopropyl-2,2-diméthyl-6-éthoxy-1,2,3,4-tétrahydroquinoléine, chlorhydrate

### Etape 1 : N-(1,1-diméthyl-2-propynyl)-N-(3,5-diisopropyl-4-éthoxyphényl)amine

Le produit attendu est obtenu selon le procédé décrit à l'étape 1 de l'exemple 1, en utilisant comme produit de départ le composé décrit dans la préparation B, et en remplaçant le 1-éthynyl-1-chlorocyclohexane par le 2-chloro-2-méthyl-3-butyne (préparé suivant la méthode décrite dans J. Am. Chem. Soc., 83, 725, 1961, à partir du 2-méthyl-3-butyn-2-ol).

### Etape 2 : 5,7-Diisopropyl-2,2-diméthyl-6-éthoxy-1,2-dihydroquinoléine

Le produit attendu est obtenu selon le procédé décrit à l'étape 2 de l'exemple 1, à partir du composé décrit à l'étape précédente.

### Etape 3 : 5,7-Diisopropyl-2,2-diméthyl-6-éthoxy-1,2,3,4-tétrahydroquinoléine, chlorhydrate

Le produit attendu est obtenu selon le procédé décrit à l'étape 3 de l'exemple 6, à partir du composé décrit au stade précédent.
*Point de fusion :* 227°-230°C (Et₂O).

| *Microanalyse élémentaire* (formule brute : C₁₉ H₃₁ N O.HCl) | | | | |
|---|---|---|---|---|
| | C | H | N | Cl |
| **% trouvé** | 70,00 | 9,68 | 4,24 | 10,82 |
| **% calculé** | 70,02 | 9,90 | 4,30 | 10,88 |

### EXEMPLE 8: 6-Ethoxy-2,2,5,7-tétramethyl-1,2,3,4-tétrahydroquinoléine, chlorhydrate

### Etape 1 : N-(1,1-Diméthyl-2-propynyl)-N-(3,5-diméthyl-4-éthoxyphényl)amine

Le produit attendu est obtenu selon le procédé décrit à l'étape 1 de l'exemple 1, en utilisant comme produit de départ, le composé décrit dans la préparation C et en remplaçant le 1-éthynyl-1-chlorocyclohexane par le 2-chloro-2-méthyl-3-butyne (préparé suivant la méthode décrite dans J. Am. Chem. Soc., 83, 725, 1961, à partir du 2-méthyl-3-butyn-2-ol).

### Etape 2 : 6 -Ethoxy-2,2,5,7-tétraméthyl-1,2-dihydroquinoléine

Le produit attendu est obtenu selon le procédé décrit à l'étape 2 de l'exemple 1, à partir du composé décrit au stade précédent.

### Etape 3 : 6-Ethoxy-2,2,5,7-tétraméthyl-1,2,3,4-tétrahydroquinoléine, chlorhydrate

Le produit attendu est obtenu selon le procédé décrit à l'étape 3 de l'exemple 6, à partir du composé décrit au stade précédent.
*Point de fusion* : 198°-200°C (Et₂O)

| *Microanalyseélémentaire* (formule brute : C₁₉ H₃₁ N O.HCl) | | | | |
|---|---|---|---|---|
| | C | H | N | Cl |
| **% trouvé** | 66,71 | 8,93 | 5,15 | 13,11 |
| **% calculé** | 66,77 | 8,97 | 5,19 | 13,35 |

### EXEMPLE 9 : 6-Ethoxy-5,7,8-triméthyl-1,2,3,4-tétrahydroquinoléine-2-spirocyclohexane, chlorhydrate

Le produit attendu est obtenu selon le procédé décrit à l'étape 3 de l'exemple 6, en utilisant comme produit de départ le composé décrit dans l'exemple 2.
*Point de fusion :* 192°-198°C (avec décomposition ; EtOH).

| *Microanalyse élémentaire* (formule brute : C₁₉ H₂₉ N O.HCl). | | | | |
|---|---|---|---|---|
| | C | H | N | Cl |
| **% trouvé** | 70,46 | 9,23 | 4,35 | 10,98 |
| **% calculé** | 70,46 | 9,34 | 4,32 | 10,95 |

### EXEMPLE 10 : 6-Ethoxy-1,2-dihydroquinoléine-2-spire-4'-pipéridine, chlorhydrate

### Etape 1 : 4-[(4-Ethoxyphényl)imino]-1-pipéridinecarboxylate de tertbutyle

A une solution de 202,6 mmol (27,8 g) de para-phénétidine et de 169 mmol (33,7 g) de N-tert-butoxycarbonyl-4-pipéridone dans 60 ml d'éther sont ajoutés 45 g de tamis moléculaire (5 Å). L'ensemble est laissé sous agitation à température ambiante pendant 15 heures. Le mélange réactionnel est alors filtré puis le filtrat concentré pour conduire au produit attendu.

### Etape 2 : 4-(4-Ethoxyaniline)-4-(2-triméthylsilyl-1-éthynyl)-1-pipéridine carboxylate de tert butyle

A une solution de 77,8 mmol (7,64 g) de triméthylsilylacétylène dans 160 ml de THF refroidie à -78°C, sont ajoutées goutte-à-goutte 66,7 mmol (42 ml) d'une solution 1,6 M de nbutyllithium en 1 h. L'ensemble est laissé sous agitation à -78°C pendant 1 h puis à température ambiante pendant 1 h supplémentaire. On additionne alors goutte-à-goutte le triméthylsilylacétylure de lithium ainsi formé à 155,5 mmol (49,5 g) du composé décrit à l'étape précédente en solution dans 500 ml de THF. Le mélange réactionnel est placé sous agitation à -78°C pendant 1 h puis 15 h à température ambiante. Le milieu réactionnel est alors versé dans un mélange d'acétate d'éthyle et de glace. La phase organique est isolée, lavée au chlorure d'ammonium (10 %), séchée puis concentrée. Le résidu huileux obtenu est purifié par chromatographie sur gel de silice en utilisant comme éluant un mélange éther de pétrole : acétate d'éthyle (4:1) pour conduire au composé attendu.

### Etape 3 : 4-(4-Ethoxyanilino)-4-(1-éthynyl)-1-pipéridine carboxylate de tert butyle

A une solution de 21,38 mmol (8,91 g) du composé décrit à l'étape précédente dans 270 ml de THF refroidie à 0°C sont ajoutées goutte-à-goutte (23,7 mmol) 24 ml d'une solution 1M de fluorure de tétrabutylammonium dans le THF. Après 1 h d'agitation à 0°C, le mélange est dilué à l'éther puis lavé à l'eau. La phase organique est séchée puis concentrée et le résidu est purifié par chromatographie sur gel de silice en utilisant comme éluant un mélange éther de pétrole : acétate d'éthyle (4:1) pour conduire au produit attendu.

### Etape 4 : 6-Ethoxy-1,2-dihydroquinoléine-2-spiro-4'-(1'-tertbutyloxycarbonylpipéridine)

Le produit attendu est obtenu selon le procédé décrit à l'étape 2 de l'exemple 1, à partir du composé décrit à l'étape précédente.

### Etape 5 : 6-Ethoxy-1,2-dihydroquinoléine-2-spiro-4'-pipéridine, chlorhydrate

A une solution de 5,99 mmol (2,06 g) du composé décrit à l'étape précédente dans 100 ml d'EtOH absolu sont ajoutés à température ambiante, 30ml d'une solution concentrée d'acide chlorhydrique. Après 1 h 30 min d'agitation les solvants sont évaporés et le résidu est repris dans un mélange acétate d'éthyle/eau. La solution biphasique est ramenée à pH=11 à l'aide d'une solution 2M de soude. La phase organique est isolée puis séchée et concentrée pour conduire au produit attendu.

Le chlorhydrate correspondant est obtenu par précipitation dans un mélange éther / dichlorométhane auquel on ajoute de l'éther chlorhydrique.

| *Microanalyse élémentaire* (formule brute : C₁₅ H₂₀ N₂ O.HCl) | | | | |
|---|---|---|---|---|
| | C | H | N | Cl |
| **% trouvé** | 63,47 | 7,58 | 9,66 | 13,58 |
| **% calculé** | 64,16 | 7,54 | 9,98 | 12,63 |

### EXEMPLE 11 : 6-Ethoxy-1,2-dihydroquinoléine-2-spire-4'-(1'-cyclopropylméthylpipéridine)chlorhydrate

A une solution de 2 mmol (0,5 g) du composé décrit dans l'exemple 10, dans 30 ml d'acétonitrile, sont ajoutées 2,45 mmol (0,23 ml) de bromométhylcyclopropane, en présence de 4 mmol (0,56 g) de carbonate de potassium. Le mélange est agité à température ambiante pendant 15 heures avant d'être filtré. Le filtrat est concentré et le résidu est purifié par chromatographie sur gel de silice en utilisant comme éluant un mélange dichlorométhane : éthanol, 9 : 1, pour conduire au produit attendu. Le chlorhydrate correspondant est obtenu par précipitation dans un mélange éther chlorhydrique / dichlorométhane.
*Point de fusion :* 170°C (avec décomposition ; Et₂O).

| *Microanalyse élémentaire* (formule brute : C₁₅ H₂₀ N₂ O.1,6 HCl) | | | | |
|---|---|---|---|---|
| | C | H | N | Cl |
| **% trouvé** | 64,36 | 7,50 | 7,86 | 16,20 |
| **% calculé** | 63,96 | 7,81 | 7,85 | 15,90 |

### EXEMPLE 12 : 2,2-Diméthyl-6-éthoxy-3-(2-morpholinoéthyl)-1,2-dihydroquinoléine, dichlorhydrate

### Etape 1 : 1-Acétyl-2,2-diméthyl-6-éthoxy-1,2-dihydroquinoléine

Une solution de 30,2 mmol (6,15 g) de 2,2-diméthyl-6-éthoxy-1,2-dihydroquinoléine (préparée selon le procédé décrit dans les étapes 1 et 2 de l'exemple 1 à partir du 2-chloro-2-méthyl-3-butyne) dans 45 ml d'anhydride acétique est chauffée à 100°C sous atmosphère inerte pendant 3 heures. Après refroidissement, le milieu est concentré et le résidu est purifié par chromatographie sur gel de silice en utilisant comme éluant un mélange dichlorométhane : acétate d'éthyle, 95 : 5, pour conduire au composé attendu.

### Etape 2 : 1-Acétyl-3-bromo-2,2-diméthyl-6-éthoxy-4-hydroxy-1,2,3,4-tétrahydroquinoléine

A une solution de 24,7 mmol (6,06 g) du composé décrit à l'étape précédente dans 154 ml d'un mélange diméthylsulfoxyde : eau, 10 : 1, refroidie à 0°C, sont ajoutées par portions 28,37 mmol (5,05 g) de N-bromosuccinimide. Le mélange réactionnel est alors dilué à l'acétate d'éthyle, puis lavé 3 fois avec 100 ml d'eau et 1 fois avec 100 ml d'une solution saturée de chlorure de sodium. La phase organique est séchée et concentrée pour conduire au composé attendu.

### Etape 3 : 1-Acétyl-3-bromo-2,2-diméthyl-6-éthoxy-1,2,3,4-tétrahydroquinoléin-4-one

A une solution de 24,51 mmol (8,39 g) du composé décrit à l'étape précédente dans 225 ml de dichlorométhane, sont ajoutés 30 g de tamis moléculaire 4 Å activé. Le milieu réactionnel est refroidi à 0°C et on y introduit par portions 31,10 mmol (23 g) de dichromate de pyridinium. Le mélange réactionnel est agité à 0°C pendant 10 minutes, puis pendant 2 heures à température ambiante. Le mélange est filtré et le précipité est rincé au dichlorométhane et à l'acétone. Le filtrat est concentré et purifié par chromatographie sur gel de silice en utilisant comme éluant un mélange éther de pétrole : acétate d'éthyle, 70 : 30, pour conduire au composé attendu.

### Etape 4 : 1-Acétyl-2,2-diméthyl-6-éthoxy-3-[(éthoxycarbonyl-tert-butoxycarbonyl) méthyl]-1,2,3, 4-tétrahydroquinoléin-4-one

A une suspension de 29,75 mmol (1,19 g) d'hydrure de sodium à 60% dans l'huile, dans 85 ml de THF refroidie à 0°C, sont ajoutées gouitte à goutte 32,35 mmol (6,09 g) de malonate de tert butyléthyle en solution dans 30 ml de THF. Apres 30 minutes d'agitation à 0°C, on introduit goutte à goutte 21,57 mmol (7,34 g) du composé décrit à l'étape précédente en solution dans 35 ml de THF. Le mélange réactionnel est agité 10 minutes à 0°C puis 3 heures à température ambiante. La réaction est neutralisée par 100 ml d'eau et le milieu est dilué à l'acétate d'éthyle. La phase organique est lavée par une solution saturée de chlorure de sodium, séchée, concentrée et purifiée par chromatographie sur gel de silice en utilisant comme éluant un mélange éther de pétrole : acétate d'éthyle , 80 : 20, pour conduire au composé attendu.

### Etape 5 : 1-Acétyl-2,2-diméthyl-6-éthoxy-3-(éthoxycarbonylméthyl)-1,2,3,4-tétrahydroquinoléin-4-one

A une solution de 5,9 mmol (2,64 g) du composé décrit à l'étape précédente dans 90 ml de dichlorométhane refroidie à 0°C, sont ajoutées 118,1 mmol (9,1 ml) d'acide trifluoroacétique. Le mélange réactionnel est agité à température ambiante pendant 15 heures. Le milieu est ensuite dilué à l'acétate d'éthyle, puis lavé par une solution saturée de NaHCO₃. La phase organique est écartée et la phase aqueuse est ramenée à pH=1 à l'aide d'une solution d'acide chlorhydrique concentrée, puis extraite à l'acétate d'éthyle. La phase organique est séchée et concentrée. Le résidu est repris dans 110 ml de dioxane, et le mélange est chauffé à reflux pendant 9 heures. Le solvant est évaporé et le résidu est purifié par chromatographie sur gel de silice en utilisant comme éluant un mélange éther de pétrole : acétate d'éthyle, 70 : 30, pour conduire au composé attendu.

### Etape 6 : 2,2-Diméthyl-6-éthoxy-4-hydroxy-3-(2-hydroxyéthyl)-1,2,3,4-tétrahydroquinoléine

A une solution de 10,05 mmol (0,96 g) du composé décrit dans l'étape précédente dans 60 ml de toluène sont ajoutés 1,50 ml d'eau et 5,05 mmol (0,96 g) d'acide p-toluènesulfonique. L'ensemble est chauffé à reflux pendant 2 heures puis les solvants sont évaporés . Le résidu est repris dans l'acétate d'éthyle et lavé par une solution saturée de NaHCO₃. La phase organique est séchée et concentrée. Le produit obtenu est mis en solution dans 15 ml de THF et additionné à 27,9 mmol (1,06 g) d'hydrure double d'aluminium et de lithium en suspension dans 100 ml de THF. Après une heure d'agitation à température ambiante, le mélange réactionnel est refroidi à l'aide d'un bain de glace et on ajoute 1 ml d'eau et 1 ml d'une solution de soude à 15%. Le mélange est ensuite filtré et le filtrat concentré pour conduire au composé attendu.

### Etape 7 : 2,2-Diméthyl-6-éthoxy-3-(2-hydroxyéthyl)-1,2-dihydroquinoléine

Une solution de 1,73 mmol (0,46 g) du composé décrit à l'étape précédente dans 1 ml de diméthylsulfoxyde est chauffée à 170°C pendant 1 heure 30 minutes. Le mélange est refroidi puis dilué à l'acétate d'éthyle. Après lavage à l'eau, la phase organique est séchée, concentrée et purifiée par chromatographie sur gel de silice en utilisant comme éluant un mélange éther de pétrole : acétate d'éthyle, 70 : 30, pour conduire au composé attendu.

### Etape 8 : 3-(2-Bromoéthyl)-2,2-diméthyl-6-éthoxy-1,2-dihydroquinoléine

A une solution de 3,68 mmol (0,91 g) du composé décrit à l'étape précédente dans 20 ml de dichlorométhane, sant ajoutées 5,73 mmol (1,9 g) de tétrabromure de carbone. L'ensemble est refroidi à 0°C et on ajoute 5,71 mmol (1,75 g) de triphénylphosphine en solution dans 15 ml de dichlorométhane. Le mélange réactionnel est agité à 0°C pendant 15 minutes et à température ambiante pendant 2 heures. Le solvant est évaporé et le résidu obtenu est purifié par chromatographie sur gel de silice en utilisant le dichlorométhane comme éluant pour conduire au composé attendu.

### Etape 9 : 2,2-Diméthyl-6-éthoxy-3-(2-morpholinoéthyl)-1,2-dihydroquinoléine, dichlorhydrate

A une solution de 0,69 mmol (0,21 g) du composé décrit à l'étape précédente dans 1 ml d'acétonitrile, sont ajoutées 2,15 mmol (0,19 g) de morpholine. Le mélange réactionnel est agité à température ambiante pendant 15 heures. Le milieu est ensuite dilué à l'acétate d'éthyle et lavé à l'eau. La phase organique est séchée, concentrée et purifiée par chromatographie sur gel de silice en utilisant comme élant un mélange dichlorométhane : éthanol, 95 : 5, pour conduire au composé attendu. Ce dernier est mis en solution dans l'acétate d'éthyle et on ajoute lentement une solution 3M d'acide chlorhydrique dans l'acétate d'éthyle. Après 15 minutes d'agitation, le solvant est évaporé et le résidu est repris dans l'isopropanol, lavé et filtré pour conduire au dichlorhydrate attendu.

| *Microanalyse élémentaire* (formule brute : C₁₉ H₂₈ N₂ O₂.2 HCl) | | | | |
|---|---|---|---|---|
| | C | H | N | Cl |
| **% trouvé** | 58,51 | 7,80 | 7,04 | 18,23 |
| **% calculé** | 58,61 | 7,77 | 7,19 | 18,21 |

### EXEMPLE 13 : 6-tert-butoxy-1,2-dihydroquinoléine-2-spirocyclohexane, chlorhydrate

### Etape 1 : N-(4-tert-butoxyphényl]-N-[1-(1-ethynyl)cyclohexyl]amine

Le produit attendu est obtenu selon le procédé décrit à l'étape 1 de l'exemple 1, à partir du composé décrit dans la préparation D.

### Etape 2 : 6-tert-butoxy-1,2-dihydroquinoléine-2-spirocyclo-hexane, chlorhydrate

Le produit attendu est obtenu selon le procédé décrit à l'étape 2 de l'exemple 1, à partir du composé décrit au stade précédent. Le chlorhydrate correspondant est obtenu par précipitation dans l'acétate d'éthyle après addition d'acétate d'éthyle chlorhydrique (3M).
*Point de fusion :* 132°C (AcOEt).

| *Microanalyse élémentaire* (formule brute : C₁₈ H₂₅ NO.HCl) | | | | |
|---|---|---|---|---|
| | C | H | N | Cl |
| **% trouvé** | 70,03 | 8,57 | 4,43 | 11,34 |
| **% calculé** | 70,23 | 8,51 | 4,55 | 11,52 |

### EXEMPLE 14 : 6-Méthoxy-1,2-dihydroquinoléine-2-spirocyclohexane, chlorhydrate

### Etape 1 : N-(2-Méthoxylphényl)-N-[1-(1-ethynyl)cyclohexyl]amine

Le produit attendu est obtenu selon le procédé décrit à l'étape 1 de l'exemple 1, en remplaçant la p-phénétidine par la p-anisidine.

### Etape 2 : 6 -Méthoxy-1,2-dihydroquinoléine-2-spirocyclohexane, chlorhydrate

Le produit attendu est obtenu selon le procédé décrit à l'étape 2 de l'exemple 1, à partir du composé décrit à l'étape précédente. Le chlorhydrate correspondant est obtenu par précipitation dans l'acétate d'éthyle après ajout d'acétate d'éthyle chlorhydrique (3M).
*Point de fusion :* 191°C (AcOEt).

| *Microanalyse élémentaire* (formule brute : C₁₅ H₁₉ NO.HCl) | | | | |
|---|---|---|---|---|
| | C | H | N | Cl |
| **% trouvé** | 67,31 | 7,59 | 5,11 | 13,42 |
| **% calculé** | 67,79 | 7,58 | 5,27 | 13,34 |

### EXEMPLE 15 : 6-Phénoxy-1,2-dihydroquinoléine-2-spirocyclohexane, chlorhydrate

### Etape 1 N-(2-Phénoxyphényl)-N-[1-(1-éthynyl)cyclohexyl]amine

Le produit attendu est obtenu selon le procédé décrit à l'étape 1 de l'exemple 1, en remplaçant la p-phénétidine par la 4-phénoxy aniline.

### Etape 2 : 6-Phénoxy-1,2-dihydroquinoléine-2-spirocyclohexane, chlorhydrate

Le produit attendu est obtenu selon le procédé décrit à l'étape 2 de l'exemple 1, à partir du composé décrit à l'étape précédente. Le chlorhydrate correspondant est obtenu par précipitation dans l'acétate d'éthyle après ajout d'acétate d'éthyle chlorhydrique (3M).
*Point de fusion :* 176°C (AcOEt).

| *Microanalyse élémentaire* (formule brute : C₂₀ H₂₁ NO.HCl) | | | | |
|---|---|---|---|---|
| | C | H | N | Cl |
| **% trouvé** | 73,36 | 6,84 | 4,43 | 10,75 |
| **% calculé** | 73,27 | 6,76 | 4,27 | 10,81 |

### EXEMPLE 16 : 6-Ethoxy-5,7-diméthyl-1,2,3,4-tétrahydroquinoléine-2-spirocyclohexane, chlorhydrate

Le produit attendu est obtenu selon le procédé décrit dans l'exemple 6, étape 3, en utilisant comme produit de départ le composé décrit dans l'exemple 5.

| *Microanalyse élémentaire* (formule brute : C₁₈ H₂₇ NO HCl) | | | | |
|---|---|---|---|---|
| | C | H | N | Cl |
| **% trouvé** | 69,27 | 9,11 1 | 4,52 | 11,44 |
| **% calculé** | 69,57 | 9,21 | 4,48 | 11,72 |

### EXEMPLE 17 : 6-Ethoxy-2,2-diméthyl-3(2-(2,6-dioxopipérazin-4-yl)éthyl]-1,2-dihydroquinoléine, chlorhydrate

Le produit attendu est obtenu selon le procédé décrit dans l'exemple 12, en remplaçant à l'étape 9, la morpholine par la 2,6-dioxopipérazine.

| *Microanalyse élémentaire* | | | | |
|---|---|---|---|---|
| | C | H | N | Cl |
| **% trouvé** | 54,81 | 6,54 | 10,09 | 17,03 |
| **% calculé** | 55,26 | 6,49 | 10,07 | 16,46 |

### EXEMPLE 18 : 6-Ethoxy-2,2-diméthyl-3[2-(1-pipéridinyl)éthyl]-1,2-dihydroquinoléine, chlorhydrate

Le produit attendu est obtenu selon le procédé décrit dans l'exemple 12, en remplaçant à l'étape 9, la morpholine par la pipérazine.

### EXEMPLE 19 : 2[2-(6-Ethoxy-2,2-diméthyl-1,2-dihydro-3-quinolinyl)éthyl]-1H-isoindole-1,3(2H)-dione, chlorhydrate

Le produit attendu est obtenu selon le procédé décrit dans l'exemple 12, en remplaçant à l'étape 9, la morpholine par le phtalimide.

### EXEMPLE 20: 3[2-(6-Ethoxy-2,2-diméthyl-1,2-dihydro-3-quinolényl)éthyl]-4(3H)-quinazolinone, chlorhydrate

Le produit attendu est obtenu selon le procédé décrit dans l'exemple 12, en remplaçant à l'étape 9, la morpholine par la 4(3H)-quinazolinone.

### EXEMPLE 21 : 6-tert-butylcarbonyloxy-1,2-dihydroquinoléine-2-spirocyclohexane, chlorhydrate

### Etape 1 N-(2-tert-butylcarbonyloxy)-N-[1-(1-éthynyl)cyclohexyl]amine

Le produit attendu est obtenu selon le procédé décrit à l'étape 1 de l'exemple 1, à partir du composé décrit dans la préparation E.

### Etape 2 : 6-tert-butylcarbonyloxy-1,2-dihydroquinoléine-2-spirocyclohexane, chlorhydrate

Le produit attendu est obtenu selon le procédé décrit à l'étape 2 de l'exemple 1, à partir du composé décrit au stade précédent. Le chlorhydrate correspondant est obtenu par précipitation dans l'acétate d'éthyle après ajout d'éther chlorhydrique (1,3M).
*Point de fusio n :* 180°C (Et₂O/AcOEt).

| *Microanalyse élémentaire* (formule brute : C₁₉ H₂₅ NO₂ HCl) | | | | |
|---|---|---|---|---|
| | C | H | N | Cl |
| **% trouvé** | 68,13 | 7,90 | 4,07 | 10,38 |
| **% calculé** | 67,94 | 7,80 | 4,17 | 10,56 |

### ETUDE PHARMACOLOGIQUE

### EXEMPLE A : Test de cytotoxicité à l'acide L-homocystéique sur cellules murines hippocampiques HT22

Des cellules hippocampiques murines HT22 en culture (100 µl/puit DMEM/F-12/25 % FCS) sont préincubées pendant 1 heure en présence de 2 concentrations (0.1 et 0.5 µM) du composé à étudier. Les cultures cellulaires sont ensuite exposées pendant 48 heures à 2 mM d'acide L-homocystéique en présence ou en absence de composé à tester. La cytotoxicité est évaluée par la méthode de réduction du 3-(4,5-diméthylthiazol-2-yl)-2,5-diphényl-tétrazolium bromide (MTT) (Mosmann T., *Immunol. Methods.* **65** : 55-63 (1983)). Les résultats sont exprimés en % de protection par rapport à la cytotoxicité mesurée dans les cultures cellulaires en absence de composé à tester.
Il apparaît que la plupart des composés de l'invention présentent un pourcentage de protection de 100 % à 0,5 µM.

### EXEMPLE B : Test de létalité au t-butylhydroperoxyde chez la souris NMRI

L'administration intracérébroventriculaire (icv) de t-butylhydroperoxyde (1 µl d'une solution à 70 %) provoque une létalité chez la souris adulte mâle NMRI (30-35 g). La létalité est mesurée 2 heures après l'administration de t-butylhydroperoxyde et est exprimée en % de protection par rapport à la létalité chez les animaux ayant reçus le véhicule des composés étudiés. Ceux-ci sont administrés à la dose de 150 mg/kg i.p. par voie intrapéritonéale 30 minutes avant l'administration de t-butylhydroperoxyde.
Il apparaît que pour la plupart des composés de l'invention le pourcentage de protection mesuré est compris entre 70 et 100 %.

### EXEMPLE C : Evaluation des effets sur la température corporelle chez la souris NMRI

La température corporelle est mesurée, à l'aide d'une sonde rectale (Physitemp, Bat-12), chez des souris adultes mâles NMRI (25-30 g), 30, 60, 90 et 120 minutes après l'administration par voie intrapéritonéale des composés étudiés à la dose de 150 mg/kg. Les résultats sont exprimés en différence de température maximale moyenne (°C) déterminée chez les animaux traités par rapport à des animaux témoins ayant reçu uniquement le véhicule (20 ml/kg).
Les résultats montrent que les composés de l'invention n'induisent pas ou peu d'effet hypothermisant à dose neuroprotectrice.

### EXEMPLE D : Antagonisme des dysfonctionnements dopaminergiques aigre-striés induits par administration de methamphetamine chez la souris C57BL/6.

Des souris mâles (C57BL/6 20-25g) reçoivent quatre injections de d-methamphetamine (5 mg/kg base, i.p.) à 2h d'intervalle (Sonsalla et Heikkila, Prog. Neuro-Psychopharmacolo. & Biol. Psychiat., 12, 345-354, 1988) et l'antioxydant testé est administré (i.p.) 30 min avant la première et la troisième injection de d-methamphetamine (Yamamoto et Zhu, J. Pharmacol. Exp. Ther. 287, 107-114, 1988). La température rectale est suivie pendant toute la durée de l'expérience. Les animaux sont sacrifiés par décapitation 72h après la dernière injection de d-methamphetamine. Les cerveaux sont rapidement extraits et les striata sont prélevés, congelés dans l'azote liquide et pesés. Les striata sont homogénéïsés par sonication dans 20 volumes d'HClO₄ 0.1 N, et l'homogénat centrifugé à 15000 g pendant 20 min à 4°C. Les surnageants sont récupérés pour le dosage des taux tissulaires striataux de dopamine réalisé par HPLC couplée à la détection coulométrique (Bonhomme et al., Brain Res., 675, 215-233, 1995). Les résultats sont exprimés en µg de dopamine/g de tissu. Il apparaît que les composés de l'invention s'opposent au déficit dopaminergique induit par l'administration de methamphetamine.

C'est notamment le cas du composé de l'exemple 1 administré à la dose de 2x 150mg/kgi.p.

### EXEMPLE E : Neuroprotection vis-à-vis d'une ischémie cérébrale globale et transitoire chez le rat Wistar

Ce modèle animal (Pulsinelli et Brierley, Stroke 10, 267-272, 1979), est couramment utilisé pour la détection d'agents anti-ischémiques centraux (Buchan et al., Neurosci. Lett., 132(2), 255-258, 1991).

Sous anesthésie au pentobarbital, les artères vertébrales de rats mâles Wistar (280-320 g, Charles River) sont définitivement occluses par électrocoagulation et des lacets carotidiens sont placés autour de chaque carotide commune. 24h plus tard, une ischémie est réalisée pendant 10 min en clampant les carotides à l'aide des lacets carotidiens. Cet épisode ischémique provoque une mort neuronale retardée au niveau des cellules pyramidales de l'hippocampe. Cette mort neuronale est mesurée par comptage neuronal sur des coupes de cerveau (7µm, coloration : hématoxyline-éosine) de rats sacrifiés 7 jours après l'épisode ischémique. Les résultats sont exprimés en pourcentage de neurones hippocampiques viables par rapport à la population neuronale hippocampique totale. Il apparaît que les composés de l'invention diminuent de façon significative la mort neuronale hippocampique consécutive à l'ischémie. C'est notamment le cas du composé de l'exemple 1 qui, administré 30 min avant le début de l'ischémie induit une diminution d'un facteur 2,7.

### EXEMPLE F : Neurodégénérescence hippocampique retardée induite par administration d'acide kaïnique chez le rat Wistar

Ce modèle est fréquemment utilisé en tant que modèle d'épilepsie temporale chez l'homme (Ben-Ari, Neurosci., 14, 375-403, 1985).

Des rats Wistar mâles (180-220 g, CERJ) reçoivent une administration, par voie sous-cutanée, d'acide kaïnique (12 mg/kg). Les animaux sont sacrifiés par décapitation 7 jours plus tard. Les cerveaux sont prélevés puis coupés en congélation en sections frontales de 7 µm qui sont colorées (hematoxyline-éosine). La mort neuronale hippocampique est objectivée par comptage neuronal au niveau de la couche CA₃ hippocampique. Les résultats sont exprimés en pourcentage de neurones viables dans la couche CA₃ par rapport à la population neuronale totale.
Il apparaît que les composés de l'invention diminuent de façon significative la mort neuronale dans la couche CA₃ de l'hippocampe. En particulier, le composé de l'exemple 1, administré à la dose de 150 mg/kg i.p., 30 min avant l'administration d'acide kaïnique induit une diminution d'un facteur 3 de la mort neuronale.

### EXEMPLE G : Composition pharmaceutique

| Formule de préparation pour 1000 comprimés dosés à 10 mg | |
|---|---|
| Composé de l'exemple 1 | 10 g |
| Hydroxypropylcellulose | 2 g |
| Amidon de blé | 10 g |
| Lactose | 100 g |
| Stéarate de magnésium | 3 g |
| Talc | 3g |

## Revendications

1. Composés de formule générale (I) : 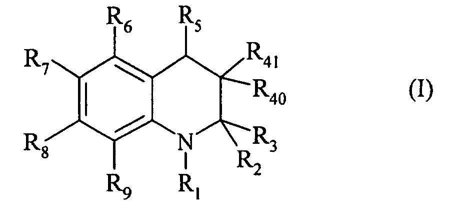 dans laquelle :
• R₁ représente un atome d'hydrogène,
• R₂ et R₃ représentent chacun un groupement alkyle,
ou bien,
R₂ et R₃ forment ensemble avec l'atome de carbone qui les porte un groupement cycloalkyle (C₃-C₈) ou un groupement hétérocycloalkyle monocyclique non substitué
ou substitué par un groupement alkyle, cycloalkyle, cycloalkylalkyle, aryle ou arylalkyle,
• R₄₀ représente un atome d'hydrogène ou un groupement -V-Q dans lequel V représente un groupement alkylène et Q représente un groupement hétérocycloalkyle,
• R₄₁ et R₅ forment ensemble une liaison, ou représentent chacun un atome d'hydrogène,
• R₆, R₇, R₈ et R₉ représentent indépendamment un atome d'hydrogène, un groupement alkyle ou un groupement -OW dans lequel W représente un groupement alkyle, acyle, ou phényle (sachant que R₆, R₇, R₈ et R₉ ne peuvent tous représenter simultanément un atome d'hydrogène et qu'au moins un d'entre eux représente un groupement -OW tel que défini précédemment),
à la condition que :
- lorsque R₂ et R₃ représentent un groupement alkyle :
• si R₆ à R₉ représentent indépendamment un atome d'hydrogène, un groupement alkyle ou un groupement -OW dans lequel W représente un groupement alkyle, tandis que R₄₁ et R₅ forment ensemble une liaison, alors R₄₀ est différent d'un atome d'hydrogène,
• le composé de formule (I) étant différent de la 6-méthoxy-2,2-diméthyl-1,2,3,4-tétrahydroquinoline,
• le composé de formule (I) étant différent de la 6,8-diméthoxy-2,2-diméthyl-1,2,3,4-tétrahydroquinoline,
étant entendu que :
- le terme alkyle désigne une chaîne de 1 à 6 atomes de carbone, linéaire ou ramifiée,
- le terme acyle désigne un groupement alkyle-carbonyle, alkyle ayant la même signification que précédemment,
- le terme alkényle désigne une chaîne de 2 à 6 atomes de carbone, linéaire ou ramifiée, contenant de 1 à 3 double(s) liaison(s),
- le terme alkynyle désigne une chaîne de 2 à 6 atomes de carbone, linéaire ou ramifiée, contenant de 1 à 3 triple(s) liaison(s),
- le terme alkylène désigne un groupement bivalent linéaire ou ramifié contenant de 1 à 6 atomes de carbone,
- le terme alkénylène désigne un groupement bivalent linéaire ou ramifié contenant de 2 à 6 atomes de carbone et de 1 à 3 doubles liaisons,
- le terme alkynylène désigne un groupement bivalent linéaire ou ramifié contenant de 2 à 6 atomes de carbone et de 1 à 3 triples liaisons,
- le terme aryle représente un groupement phényle, naphtyle ou biphényle,
- le terme hétérocycloalkyle désigne un groupement mono ou bicyclique de 4 à 11 chaînons contenant de 1 à 6 hétéroatomes choisis parmi azote, oxygène et soufre, ce groupement pouvant comporter une ou plusieurs insaturations sans pour autant présenter un caractère aromatique,
- le terme substitué affecté aux expressions aryle, et arylalkyle, signifie que les groupements concernés sont substitués par un ou plusieurs atomes d'halogène, ou groupements alkyle, alkoxy (C₁-C₆) linéaire ou ramifié, perhalogénoalkyle (C₁-C₆) linéaire ou ramifié, amino (éventuellement substitué par 1 ou 2 groupements alkyle), cyano, carboxy, alkoxycarbonyl (C₁-C₆) linéaire ou ramifié, aminocarbonyle (éventuellement substitué sur l'atome d'azote par 1 ou 2 groupements alkyle), nitro, ou hydroxy,
- le terme substitué affecté aux expressions alkyle, alkényle, alkynyle, et cycloalkyle, signifie que ces groupements sont substitués par un ou plusieurs groupements choisis parmi hydroxy, alkoxy (C₁-C₆) linéaire ou ramifié, alkylthio (C₁-C₆) linéaire ou ramifié, amino (éventuellement substitué par un ou deux groupements alkyle), carboxy, nitro, cyano, alkoxycarbonyl (C₁-C₆) linéaire ou ramifié, aminocarbonyle (éventuellement substitué sur l'atome d'azote par un ou deux groupements alkyle),
- le terme substitué affecté aux expressions hétérocycloalkyle,
- hétérocycloalkylalkyle, signifie que les groupements concernés sont substitués par un ou plusieurs atomes d'halogène, ou groupements alkyle, alkoxy (C₁-C₆) linéaire ou ramifié, perhalogénoalkyle (C₁-C₆) linéaire ou ramifié, amino (éventuellement substitué par 1 ou 2 groupements alkyle), cyano, carboxy, alkoxycarbonyl (C₁-C₆) linéaire ou ramifié, aminocarbonyle (éventuellement substitué sur l'atome d'azote par 1 ou 2 groupements alkyle), nitro, hydroxy, ou oxo,
leurs énantiomères, diastéréoisomères, ainsi que leurs sels d'addition à un acide ou à une base pharmaceutiquement acceptable.

2. Composés de formule (I) selon la revendication 1 pour lesquels R₁ représente un atome d'hydrogène, R₂ et R₃ représentent un groupement alkyle ou forment ensemble un groupement cycloalkyle, R₄₀ représente un atome d'hydrogène ou un groupement -V-Q dans lequel V représente un groupement alkylène et Q représente un groupement hétérocycloalkyle, et R₆, R₇, R₈, R₉ représentent indépendamment un atome d'hydrogène, un groupement alkyle, ou un groupement -OW dans lequel W représente un groupement alkyle, acyle ou phényle, sachant que R₆, R₇, R₈ et R₉ ne peuvent tous représenter un atome d'hydrogène et qu'au moins un d'entre eux représente un groupement -OW tel que défini précédemment, leurs énantiomères, diastéréoisomères, ainsi que leurs sels d'addition à un acide ou à une base pharmaceutiquement acceptable.

3. Composés de formule (I) selon la revendication 2 pour lesquels R₂ et R₃ forment ensemble un groupement cycloalkyle, leurs énantiomères, diastéréoisomères, ainsi que leurs sels d'addition à un acide ou à une base pharmaceutiquement acceptable.

4. Composés de formule (I) selon la revendication 2 pour lesquels R₂ et R₃ représentent un groupement alkyle, leurs énantiomères, diastéréoisomères, ainsi que leurs sels d'addition à un acide ou à une base pharmaceutiquement acceptable.

5. Composé de formule (I) selon la revendication 1 qui est le 6-éthoxy-2,2,5,7,8-pentaméthyl-1,2,3,4-tétrahydroquinoléine, ainsi que ses sels d'addition à un acide pharmaceutiquement acceptable.

6. Composé de formule (I) selon la revendication 1 qui est le 6-éthoxy-1,2-dihydroquinoléine-2-spirocyclohexane, ainsi que ses sels d'addition à un acide pharmaceutiquement acceptable.

7. Procédé de préparation des composés de formule (I), **caractérisé en ce que** l'on utilise comme produit de départ une aniline diversement substituée de formule (II) : 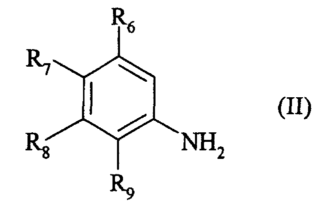 dans laquelle R₆, R₇, R₈, R₉, sont tels que définis dans la formule (I),
qui est soumis :
• soit à l'action, en milieu basique et éventuellement en présence d'un catalyseur, d'un acétylénure halogéné de formule (III) : 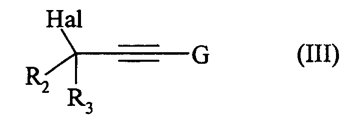 dans laquelle R₂ et R₃ sont tels que définis dans la formule (I), G représente un atome d'hydrogène ou un groupement trialkylsilyle, et Hal représente un atome d'halogène,
pour conduire à un composé de formule (IV) : 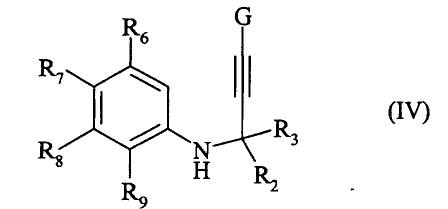 dans laquelle R₂, R₃, R₆, R₇, R₈, R₉ et G sont tels que définis précédemment,
• soit à l'action d'un dérivé carbonylé de formule (V) : 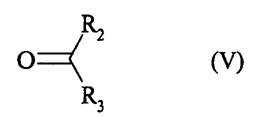 dans laquelle R₂ et R₃ sont tels que définis dans la formule (I),
pour conduire à un composé de formule (VI) : 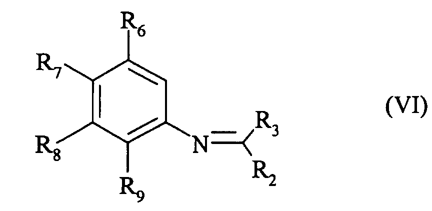 dans laquelle R₂, R₃, R₆, R₇, R₈ et R₉ sont tels que définis précédemment,
composé (VI) qui est soumis, en milieu basique, à l'action d'un acétylénure de formule (III) telle que définie précédemment,
pour conduire à un composé de formule (IV) telle que précédemment définie,
composé de formule (IV), qui après coupure éventuelle du groupement trialkylsilyle, est cyclisé par chauffage en présence d'un catalyseur approprié, pour conduire à un composé de formule (I/a) : 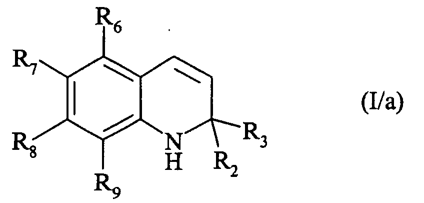 cas particulier des composés de formule (I) pour lequel R₂, R₃, R₆, R₇, R₈ et R₉, sont tels que définis précédemment,
qui peut être :
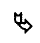 soit soumis à une réaction de réduction pour conduire à un composé de formule (I/b) : 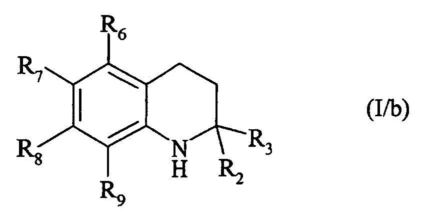 cas particulier des composés de formule (I) pour lequel R₂, R₃, R₆, R₇, R₈ et R₉, sont tels que définis précédemment,
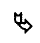 soit, après protection de l'atome d'azote cyclique, soumis successivement à une réaction d'hydroxyhalogénation et à une réaction d'oxydation en position benzylique, pour conduire à un composé de formule (VIII) : 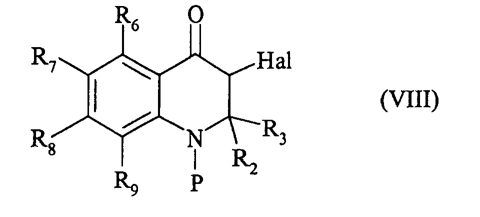
dans laquelle R₂, R₃, R₆, R₇, R₈, R₉ sont tels que définis précédemment, Hal représente un atome d'halogène, et P est un groupement protecteur de l'azote cyclique (par exemple un groupement acétyle, trifluoroacétyle, tertbutoxycarbonyle ou benzyloxycarbonyle),
qui est soumis à une réaction de substitution nucléophile pour conduire à un composé de formule (IX) : 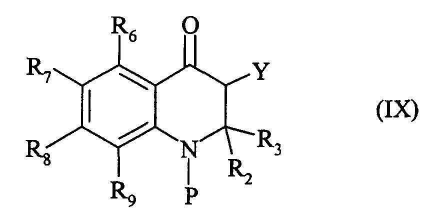 dans laquelle R₂, R₃, R₆, R₇, R₈, R₉ et P sont tels que définis précédemment, et Y représente soit un groupement R₄₀ différent d'un atome d'hydrogène tel que défini dans la formule (I), soit un précurseur de ce groupement,
qui, après déprotection de l'azote cyclique, subit une réaction de réduction de la fonction carbonyle, suivie d'une élimination, pour conduire à un composé de formule (X) : 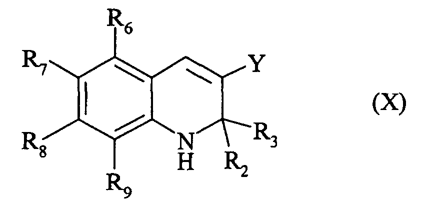 dans laquelle R₂, R₃, R₆, R₇, R₈, R₉ et Y sont tels que définis précédemment,
composé de formule (X) qui peut, lorsque Y est un précurseur d'un groupement R₄₀ tel que défini précédemment, subir une suite de réactions classiques dans le but de conduire à un composé de formule (I/c) : 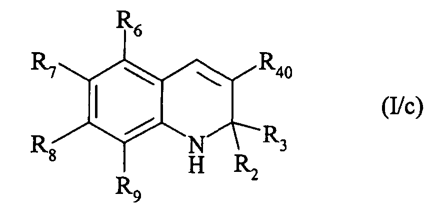 cas particulier des composés de formule (I) pour lequel R₂, R₃, R₆, R₇, R₈, R₉ et R₄₀ sont tels que définis précédemment,
qui peut être réduit pour conduire à un composé de formule (I/d): 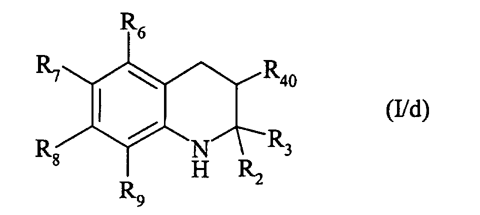 cas particulier des composés de formule (I) pour lequel R₂, R₃, R₄₀, R₆, R₇, R₈ et R₉ sont tels que définis précédemment,
composés (I/a) à (I/d), formant la totalité des composés de formule (I) et :
- qui peuvent être, le cas échéant, purifiés selon une technique classique de purification,
- dont on sépare, le cas échéant, les stéréoisomères selon une technique classique de séparation,
- que l'on transforme, si on le souhaite, en leurs sels d'addition à un acide ou à une base pharmaceutiquement acceptable.

8. Compositions pharmaceutiques contenant comme principe actif au moins un composé selon l'une quelconque des revendications 1 à 6, seul ou en combinaison avec un ou plusieurs excipients ou véhicules inertes, non toxiques, pharmaceutiquement acceptables.

9. Compositions pharmaceutiques selon la revendication 8 contenant au moins un principe actif selon l'une quelconque des revendications 1 à 6 utiles comme agents antioxydants dans le traitement d'affections liées à la sénescence, comme l'athérosclérose ou la cataracte, dans le traitement des troubles cognitifs, dans le traitement des maladies neurodégénératives aiguës telles que l'ischémie cérébrale et l'épilepsie et dans le traitement des maladies neurodégénératives chroniques telles que la maladie d'Alzheimer, la maladie de Pick ou les neurodégénérescences des ganglions de la base (maladie de Parkinson, Hungtington).

## Patentansprüche

1. Verbindungen der allgemeinen Formel (I) : 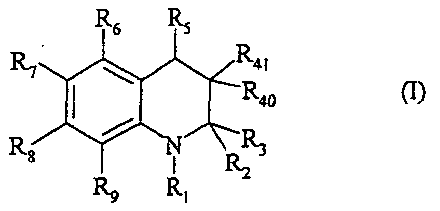 in der:
• R₁ ein Wasserstoffatom darstellt,
• R₂ und R₃ jeweils eine Alkylgruppe bedeuten,
oder
R₂ und R₃ gemeinsam mit dem sie tragenden Kohlenstoffatom eine (C₃-C₈)-Cycloalkylgruppe oder eine monocyclische nichtsubstituierte oder durch eine Alkyl-, Cycloalkyl-, Cycloalkylalkyl-, Aryl- oder Arylalkylgruppe substituierte Heterocycloalkylgruppe bilden,
• R₄₀ ein Wasserstoffatom oder eine Gruppe -V-Q darstellt, worin V eine Alkylengruppe und Q eine Heterocycloalkylgruppe bdeuten,
• R₄₁ und R₅ gemeinsam eine Bindung bilden oder jeweils ein Wasserstoffatom darstellen,
• R₆, R₇, R₈ und R₉ unabhängig voneinander jeweils ein Wasserstoffatom, eine Alkylgruppe oder eine Gruppe -OW, worin W eine Alkyl-, Acyl- oder Phenylgruppe darstellt, bedeuten (mit der Maßgabe, daß R₆, R₇, R₈ und R₉ nicht sämtlich gleichzeitig ein Wasserstoffatom bedeuten können und daß mindestens eine dieser Gruppen eine Gruppe -OW darstellt, wie sie oben definiert worden ist),
mit der Maßgabe, daß:
- wenn R₂ und R₃ eine Alkylgruppe darstellen:
• wenn R₆ bis R₉ unabhängig voneinander ein Wasserstoffatom, eine Alkylgruppc oder eine Gruppe -OW bedeuten, worin W eine Alkylgruppe darstellt, während R₄₁ und R₅ gemeinsam eine Bindung bilden, dann R₄₀ von einem Wasserstoffatom verschieden ist,
• die Verbindung der Formel (I) von 6-Methoxy-2,2-dimethyl-1,2,3,4-tetrahydrochinolin verschieden ist,
• die Verbindung der Formel (I) von 6,8-Dimethoxy-2,2-dimethyl-1,2,3,4-tetrahydrochinolin verschieden ist,
mit der Maßgabe, daß:
- der Begriff Alkyl für eine geradkettige oder verzweigte Kette mit 1 bis 6 Kohlenstoffatomen steht,
- der Begriff Acyl für eine Alkylcarbonylgruppe steht, worin Alkyl die oben angegebene Bedeutung besitzt,
- der Begriff Alkenyl für eine geradkettige oder verzweigte Kette mit 2 bis 6 Kohlenstoffatomen steht, die 1 bis 3 Doppelbindungen aufweist,
- der Begriff Alkinyl für eine geradkettige oder verzweigte Kette mit 2 bis 6 Kohlenstoffatomen steht, die 1 bis 3 Dreifachbindungen enthält,
- der Begriff Alkylen für eine geradkettige oder verzweigte zweiwertige Gruppe steht, die 1 bis 6 Kohlenstoffatome enthält,
- der Begriff Alkenylen für eine geradkettige oder verzweigte zweiwertige Gruppe steht, die 2 bis 6 Kohlenstoffatome und 1 bis 3 Doppelbindungen enthält,
- der Begriff Alkinylen für eine geradkettige oder verzweigte zweiwertige Grppe steht, die 2 bis 6 Kohlenstoffatome und 1 bis 3 Dreifachbindungen enthält,
- der Begriff Aryl für eine Phenyl-, Naphthyl- oder Biphenylgruppe steht,
- der Begriff Heterocycloalkyl für eine mono- oder bicyclische Gruppe mit 4 bis 11 Kettengliedern steht, die 1 bis 6 Heteroatome ausgewählt aus Stickstoff, Sauerstoff und Schwefel enthält, wobei diese Gruppe eine oder mehrere Unsättigungen aufweist, ohne einen aromatischen Charakter zu besitzen,
- der Begriff substituiert, bezogen auf die Begriffe Aryl und Arylalkyl bedeutet, daß die betreffenden Gruppen durch eines oder mehrere Halogenatome oder Alkylgruppen, geradkettige oder verzweigte (C₁-C₆-Alkoxygruppen, geradkettige oder verzweigte (C₁-C₆)-Perhalogenalkylgruppen, Aminogruppen (die gegebenenfalls durch 1 oder 2 Alkylgruppen substituiert sind), Cyanogruppen, Carboxygruppen, geradkettige oder verzweigte (C₁-C₆)-Alkoxycarbonylgruppen, Aminocarbonylgruppen (die gegebenenfalls am Stickstoff durch 1 oder 2 Alkylgruppen substituiert sind), Nitrogruppen oder Hydroxygruppen substituiert sind,
- der Begriff substituiert, bezogen auf die Begriffe Alkyl, Alkenyl, Alkinyl und Cycloalkyl bedeutet, daß diese Gruppen durch eine oder mehrere Gruppen ausgewählt aus Hydroxy, geradkettigem oder verzweigtem (C₁-C₆)-Alkoxy, geradkettigem oder verzweigtem (C₁-C₆)-Alkylthio, Amino (gegebenenfalls durch eine oder mehrere Alkylgruppen substituiert), Carboxy, Nitro, Cyano, geradkettigem oder verzweigtem (C₁-C₆)-Alkoxycarbonyl, Aminocarbonyl (gegebenenfalls substituiert am Stickstoffatom durch eine oder zwei Alkylgruppen) substituiert sind,
- der Begriff substituiert, bezogen auf die Begriffe Heterocycloalkyl und Heterocycloalkylalkyl bedeutet, daß die betreffenden Gruppen durch ein oder mehrere Halogenatome oder Alkylgruppen, geradkettige oder verzweigte (C₁-C₆)-Alkoxygruppen, geradkettige oder verzweigte (C₁-C₆)-Perhalogenalkylgruppen, Aminogruppen (gegebenenfalls durch eine oder zwei Alkylgruppen substituiert), Cyanogruppen, Carboxygruppen, geradkettige oder verzweigte (C₁-C₆)-Alkoxycarbonylgruppen, Aminocarbonylgruppen (gegebenenfalls am Stickstoffatom substituiert durch eine oder zwei Alkylgruppen), Nitrogruppen, Hydroxygruppen oder Oxogruppen substituiert sind,
deren Enantiomere, Diastereoisomere sowie deren Additionssalze mit einer pharmazeutisch annehmbaren Säure oder Base.

2. Verbindungen der Formel (I) nach Anspruch 1, worin R₁ ein Wasserstoffatom, R₂ und R₃ eine Alkylgruppe oder gemeinsam eine Cycloalkylgruppe, R₄₀ ein Wasserstoffatom oder eine Gruppe -V-Q, worin V eine Alkylengruppe und Q eine Heterocycloalkylgruppe darstellen, und R₆, R₇, R₈ und R₉ unabhängig voneinander ein Wasserstoffatom, eine Alkylgruppe oder eine Gruppe -OW, worin W eine Alkylgruppe, Acylgruppe oder Phenylgruppe darstellt, bedeuten, mit der Maßgabe, daß R₆, R₇, R₈ und R₉ nicht sämtlich ein Wasserstoffatom darstellen und mindestens eine dieser Gruppen eine Gruppe -OW darstellt, wie sie oben definiert wordenist, deren Enantiomere, Diastereoisomere sowie deren Additionssalze mit einer pharmazeutisch annehmbaren Säure oder Base.

3. Verbindungen der Formel (I) nach Anspruch 2, worin R₂ und R₃ gemeinsam eine Cycloalkylgruppe bilden, deren Enantiomere, Diastereoisomere sowie deren Additionssalze mit einer pharmazeutisch annehmbaren Säure oder Base.

4. Verbindungen der Formel (I) nach Anspruch 2, worin R₂ und R₃ eine Alkylgruppe bedeuten, deren Enantiomere, Diastereoisomere sowie deren Additionssalze mit einer pharmazeutisch annehmbaren Säure oder Base.

5. Verbindung der Formel (I) nach Anspruch 1, nämlich 6-Ethoxy-2,2,5,7,8-pentamethyl-1,2,3,4-tetrahydrochinolin, sowie dessen Additionssalze mit einer pharmazeutisch annehmbaren Säure.

6. Verbindung der Formel (I) nach Anspruch 1, nämlich 6-Ethoxy-1,2-dihydrochinolin-2-spirocyclohexan. sowie dessen Additionssalze mit einer pharmazeutisch annehmbaren Säure.

7. Verfahren zur Herstellung der Verbindungen der Formel (I), **dadurch gekennzeichnet, daß** man als Ausgangsprodukt ein unterschiedlich substituiertes Anilin der Formel (II) verwendet: 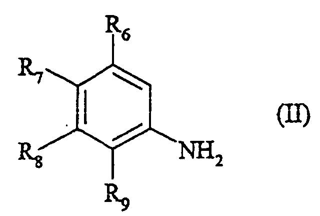 in der R₆, R₇, R₈ und R₉ die bezüglich der Formel (I) angegebenen Bedeutungen besitzen,
welches man:
• entweder in basischem Medium und gegebenenfalls in Gegenwart eines Katalysators der Einwirkung eines Halogenacetylenids der Formel (III) unterwirft: 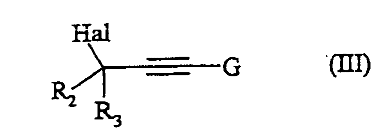 in der R₂ und R₃ die bezüglich der Formel (I) angegebenen Bedeutungen besitzen, G ein Wasserstoffatom oder eine Trialkylsilylgruppe darstellt und Hal ein Halogenatom bedeutet,
zur Bildung einer Verbindung der Formel (IV): 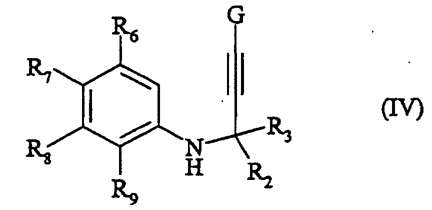 in der R₂, R₃, R₆, R₇, R₈, R₉ und G die oben angegebenen Bedeutungen besitzen,
• oder der Einwirkung eines Carbonylderivats der Formel (V) unterwirft: 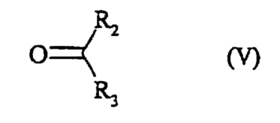 in der R₂ und R₃ die bezüglich der Formel (I) angegebenen Bedeutungen besitzen,
zur Bildung einer Verbindung der Formel (VI): 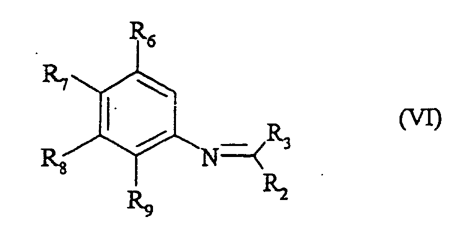 in der R₂, R₃, R₆, R₇, R₈ und R₉ die oben angegebenen Bedeutungen besitzen,
welche Verbindung (VI) in basischem Medium der Einwirkung eines Acetylenids der Formel (III), wie sie oben definiert worden ist, unterworfen wird, zur Bildung einer Verbindung der oben definierten Formel (IV),
welche Verbindung der Formel (IV) nach der eventuellen Abspaltung der Trialkylsilylgruppe durch Erhitzen in Gegenwart eines geeigneten Katalysators cyclisiert wird zur Bildung einer Verbindung der Formel (I/a): 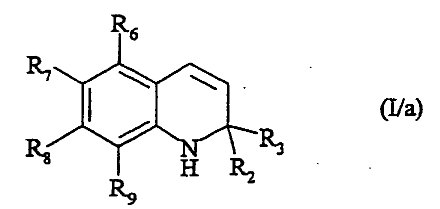 einem Sonderfall der Verbindungen der Formel (I), worin R₂, R₃, R₆, R₇, R₈ und R₉ die oben angegebenen Bedeutungen besitzen,
welche:
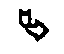 entweder einer Reduktionsreaktion unterworfen wird zur Bildung einer Verbindung der Formel (I/b): 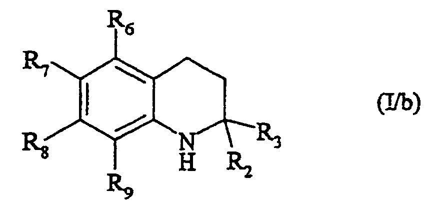 einem Sonderfall der Verbindungen der Formel (I), worin R₂, R₃, R₆, R₇, R₈ und R₉ die oben angegebenen Bedeutungen besitzen,
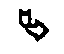 oder nach dem Schutz des cyclischen Stickstoffatoms nacheinander einer Hydroxyhalogenierungsreaktion und einer Oxidationsreaktion an der Benzyi-Position unterworfen wird zur Bildung einer Verbindung der Formel (VIII): 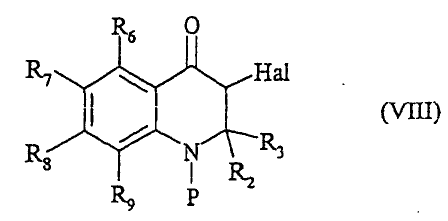 in der R₂, R₃, R₆, R₇, R₈ und R₉ die oben angegebenen Bedeutungen besitzen, Hal ein Halogenatom darstellt und P eine Schutzgruppe für den cyclischen Stickstoff (beispielsweise eine Acetyl-, Trifluoracetyl-, tert.-Butoxycarbonyl- oder Benzyloxycarbonylgruppe) darstellt,
welche einer nucleophilen Substitutionsreaktion unterworfen wird zur Bildung einer Verbindung der Formel (IX): 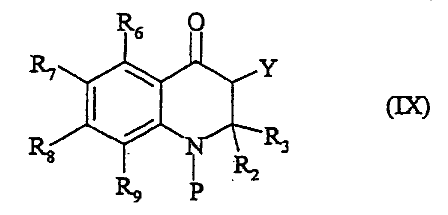 in der R₂, R₃, R₆, R₇, R₈, R₉ und P die oben angegebenen Bedeutungen besitzen und Y entweder eine von einem Wasserstoffatom verschiedene Gruppe R₄₀, wie sie oben bezüglich der Formel (I) definiert worden ist, der einen Vorläufer dieser Gruppe darstellt,
welche nach der Abspaltung der Schutzgruppe des cyclischen Stickstoffatoms einer Reduktionsreaktion der Carbonylgruppe unterworfen wird, gefolgt von einer Eliminierung zur Bildung eine Verbindung der Forme (X): 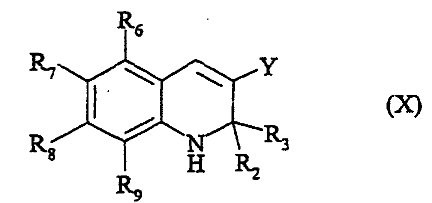 in der R₂, R₃, R₆, R₇, R₈, R₉ und Y die oben angegebenen Bedeutungen besitzen,
welche Verbindung der Formel (X) dann, wenn Y einen Vorläufer einer Gruppe R₄₀, wie sie oben definiert worden ist, darstellt, einer Folge von klassischen Reaktionen unterworfen werden kann mit dem Ziel, eine Verbindung der Formel (I/c) zu ergeben: 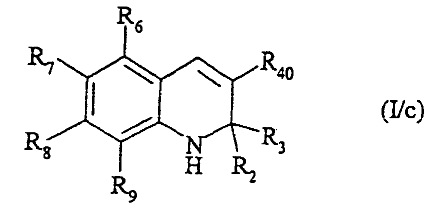 einem Sonderfall der Verbindungen der Formel (I), worin R₂, R₃, R₆, R₇, R₈, R₉ und R₄₀ die oben angegebenen Bedeutungen besitzen,
welche reduziert werden kann zur Bildung einer Verbindung der Formel (I/d): 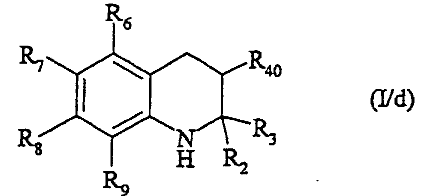 einem Sonderfall der Verbindungen der Formel (I), worin R₂, R₃, R₄₀, R₆, R₇, R₈ und R₉ die oben angegebenen Bedeutungen besitzen,
welche Verbindungen (I/a) bis (I/d) die Gesamtheit der Verbindungen der Formel (I) bilden und:
- welche gegebenenfalls mit Hilfe einer klassischen Reinigungsmethode gereinigt werden können,
- welche man gegebenenfalls mit Hilfe einer klassischen Trennmethode in ihre Stereoisomeren trennen kann und
- welche man gewünschtenfalls in ihre Additionssalze mit einer pharmazeutisch annehmbaren Säure oder Base übeführt.

8. Pharmazeutische Zubereitungen enthaltend als Wirkstoff mindestens eine Verbindung nach einemd er Ansprüche 1 bis 6 allein oder in Kombination mit einem oder mehreren inerten nichttoxischen, pharmazeutisch annehmbaren Trägermaterialien oder Bindemitteln.

9. Pharmazeutische Zubereitungen nach Anspruch 8 enthaltend mindestens einen Wirkstoff nach einem der Ansprüche 1 bis 6 als antioxidierende Mittel bei der Behandlung von mit dem Altern verknüpften Erkrankungen, wie der Atherosklerose oder dem Katarakt, bei der Behandlung von Erkenntnisstörungen, bei der Behandlung von akuten neurodegenerativen Erkrankungen, wie der zerebralen Ischämie und der Epilepsie und bei der Behandlung von chronischen neurodegenerativen Erkrankungen, wie der Alzheimerschen Krankheit, der Pickschen Krankheit oder der Nervergewebezerstörung von Grundganglien (Parkinsonschen Krankheit und Huntington-Krankheit).

## Claims

1. Compounds of the general formula (I) : 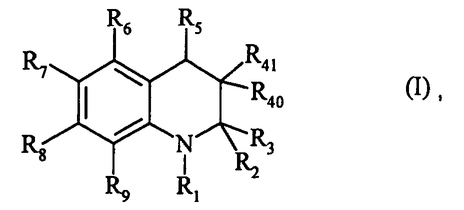 wherein:
• R₁ represents a hydrogen atom,
• R₂ and R₃ each represents an alkyl group
or
R₂ and R₃, together with the carbon atom carrying them, form a (C₃-C₈)cycloalkyl group or a monocyclic heterocycloalkyl group unsubstituted or substituted by an alkyl, cycloalkyl, cycloalkylalkyl, aryl or arylalkyl group,
• R₄₀ represents a hydrogen atom or a group -V-Q wherein V represents an alkylene group and Q represents a heterocycloalkyl group,
• R₄₁ and R₅ together form a bond or each represents a hydrogen atom,
• R₆, R₇, R₈ and R₉ each independently represents a hydrogen atom, an alkyl group or a group -OW wherein W represents an alkyl group, an acyl group or a phenyl group (provided that R₆, R₇, R₈ and R₉ cannot all simultaneously represent a hydrogen atom and that at least one of them represents a group -OW as defined hereinbefore),
with the proviso that:
- when R₂ and R₃ represent an alkyl group :
• if each of R₆ to R₉ independently represents a hydrogen atom, an alkyl group or a group -OW wherein W represents an alkyl group, and R₄₀ and R₅ together form a bond, then R₄₀ is other than a hydrogen atom,
• the compound of formula (I) being other than 6-methoxy-2,2-dimethyl-1,2,3,4-tetrahydroquinoline,
• the compound of formula (I) being other than 6,8-dimethoxy-2,2-dimethyl-1,2,3,4-tetrahydroquinoline,
it being understood that:
- the term alkyl denotes a linear or branched chain of from 1 to 6 carbon atoms,
- the term acyl denotes an alkyl-carbonyl group, alkyl being as defined hereinbefore,
- the term alkenyl denotes a linear or branched chain of from 2 to 6 carbon atoms containing from 1 to 3 double bond(s),
- the term alkynyl denotes a linear or branched chain of from 2 to 6 carbon atoms containing from 1 to 3 triple bond(s),
- the term alkylene denotes a linear or branched bivalent group containing from 1 to 6 carbon atoms,
- the term alkenylene denotes a linear or branched bivalent group containing from 2 to 6 carbon atoms and from 1 to 3 double bonds,
- the term alkynylene denotes a linear or branched bivalent group containing from 2 to 6 carbon atoms and from 1 to 3 triple bonds,
- the term aryl denotes a phenyl, naphthyl or biphenyl group,
- the term heterocycloalkyl denotes a mono- or bi-cyclic, 4- to 11-membered group containing from 1 to 6 hetero atoms selected from nitrogen, oxygen and sulphur, it being possible for the group to contain one or more unsaturations without thereby having an aromatic character,
- the term substituted used in respect of the expressions aryl and arylalkyl indicates that the groups concerned are substituted by one or more halogen atoms or alkyl, linear or branched (C₁-C₆)alkoxy, linear or branched (C₁-C₆)perhaloalkyl, amino (optionally substituted by 1 or 2 alkyl groups), cyano, carboxy, linear or branched (C₁-C₆)alkoxycarbonyl, aminocarbonyl (optionally substituted on the nitrogen atom by 1 or 2 alkyl groups), nitro or hydroxy groups,
- the term substituted used in respect of the expressions alkyl, alkenyl, alkynyl and cycloalkyl indicates that such groups are substituted by one or more groups selected from hydroxy, linear or branched (C₁-C₆)alkoxy, linear or branched (C₁-C₆)alkylthio, amino (optionally substituted by one or two alkyl groups), carboxy, nitro, cyano, linear or branched (C₁-C₆)alkoxycarbonyl and aminocarbonyl (optionally substituted on the nitrogen atom by one or two alkyl groups),
- the term substituted used in respect of the expressions heterocycloalkyl and heterocycloalkylalkyl indicates that the groups concerned are substituted by one or more halogen atoms or alkyl, linear or branched (C₁-C₆)alkoxy, linear or branched (C₁-C₆)perhaloalkyl, amino (optionally substituted by 1 or 2 alkyl groups), cyano, carboxy, linear or branched (C₁-C₆)alkoxycarbonyl, aminocarbonyl (optionally substituted on the nitrogen atom by 1 or 2 alkyl groups), nitro, hydroxy or oxo groups,
enantiomers and diastereoisomers thereof, and addition salts thereof with a pharmaceutically acceptable acid or base.

2. Compounds of formula (I) according to claim 1, wherein R₁ represents a hydrogen atom, R₂ and R₃ represent an alkyl group or together form a cycloalkyl group, R₄₀ represents a hydrogen atom or a group -V-Q wherein V represents an alkylene group and Q represents a heterocycloalkyl group, and R₅, R₇, R₈ and R₉ each independently represents a hydrogen atom, an alkyl group or a group -OW wherein W represents an alkyl, acyl or phenyl group, it being understood that R₆, R₇, R₈ and R₉ cannot all represent a hydrogen atom and that at least one of them represents a group -OW as defined hereinbefore, enantiomers and diastereoisomers thereof, and addition salts thereof with a pharmaceutically acceptable acid or base.

3. Compounds of formula (I) according to claim 2, wherein R₂ and R₃ together form a cycloalkyl group, enantiomers and diastereoisomers thereof, and addition salts thereof with a pharmaceutically acceptable acid or base.

4. Compounds of formula (I) according to claim 2, wherein R₂ and R₃ represent an alkyl group, enantiomers and diastereoisomers thereof, and addition salts thereof with a pharmaceutically acceptable acid or base.

5. Compound of formula (I) according to claim 1 which is 6-ethoxy-2,2,5,7,8-pentamethyl-1,2,3,4-tetrahydroquinoline, and addition salts thereof with a pharmaceutically acceptable acid.

6. Compound of formula (I) according to claim 1 which is 6-ethoxy-1,2-dihydroquinoline-2-spirocyclohexane, and addition salts thereof with a pharmaceutically acceptable acid.

7. Process for the preparation of compounds of formula (I), **characterised in that** there is used as starting material a variously substituted aniline of formula (II) : 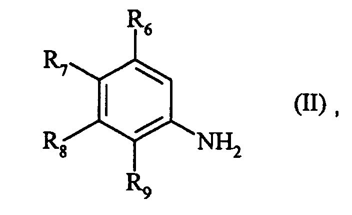 wherein R₆, R₇, R₈ and R₉ are as defined for formula (I),
which is subjected:
• either to the action, in a basic medium and optionally in the presence of a catalyst, of a halogenated acetylide of formula (III): 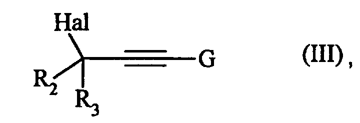 wherein R₂ and R₃ are as defined in formula (I), G represents a hydrogen atom or a trialkylsilyl group and Hal represents a halogen atom,
to yield a compound of formula (IV) : 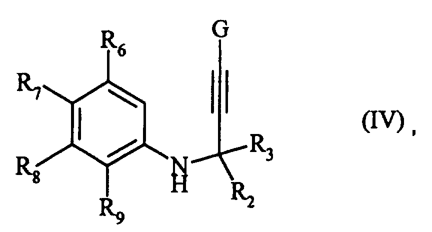 wherein R₂, R₃, R₆, R₇, R₈, R₉ and G are as defined hereinbefore,
• or to the action of a carbonyl compound of formula (V) : 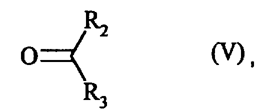 wherein R₂ and R₃ are as defined for formula (I),
to yield a compound of formula (VI) : 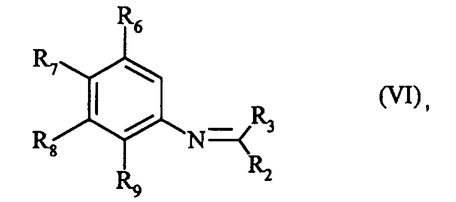 wherein R₂, R₃, R₆, R₇, R₈ and R₉ are as defined hereinbefore,
which compound (VI) is subjected, in a basic medium, to the action of an acetylide of formula (III) as defined hereinbefore,
to yield a compound of formula (IV) as defined hereinbefore,
which compound of formula (IV), where applicable after cleavage of the trialkylsilyl group, is cyclised by heating in the presence of an appropriate catalyst to yield a compound of formula (I/a) : 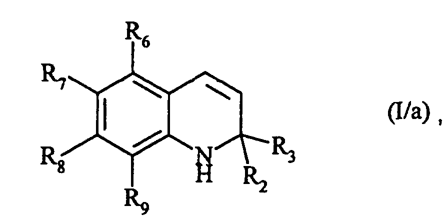 a particular case of the compounds of formula (I) wherein R₂, R₃, R₆, R₇, R₈ and R₉ are as defined hereinbefore,
which :
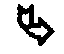 either may be subjected to a reduction reaction to yield a compound of formula (I/b) : 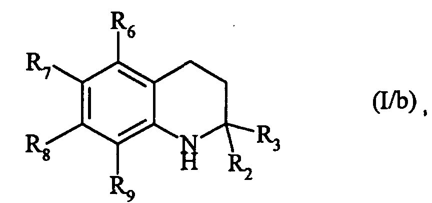 a particular case of the compounds of formula (I) wherein R₂, R₃, R₆, R₇, R₈ and R₉ are as defined hereinbefore,
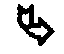 or, after protection of the ring nitrogen atom, may be subjected successively to a hydroxyhalogenation reaction and to an oxidation reaction in the benzyl position to yield a compound of formula (VIII) : 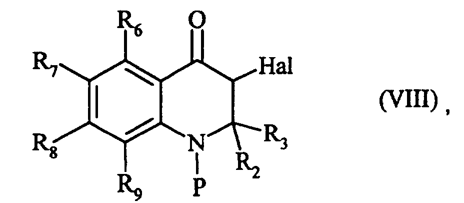 wherein R₂, R₃, R₆, R₇, R₈ and R₉ are as defined hereinbefore, Hal represents a halogen atom and P is a protecting group for the ring nitrogen (for example an acetyl, trifluoroacetyl, tert-butoxycarbonyl or benzyloxycarbonyl group),
which is subjected to a nucleophilic substitution reaction to yield a compound of formula (IX) : 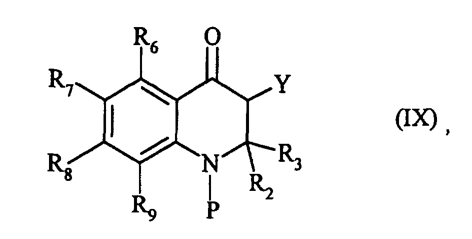 wherein R₂, R₃, R₆, R₇, R₈, R₉ and P are as defined hereinbefore and Y represents either a group R₄₀, which is as defined for formula (I) but is other than a hydrogen atom, or a precursor of such a group,
which, after deprotection of the ring nitrogen, is subjected to a reduction reaction of the carbonyl function, followed by an elimination reaction, to yield a compound of formula (X) : 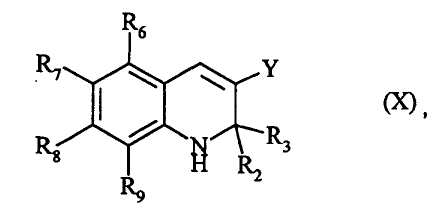 wherein R₂, R₃, R₆, R₇, R₈, R₉ and Y are as defined hereinbefore,
which compound of formula (X) may, when Y is a precursor of a group R₄₀ as defined hereinbefore, be subjected to a succession of conventional reactions directed at yielding a compound of formula (I/c) : 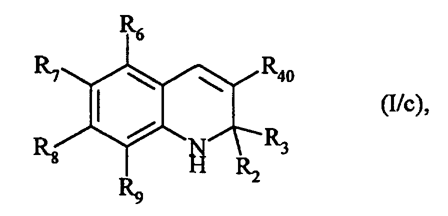 a particular case of the compounds of formula (I) wherein R₂, R₃, R₆, R₇, R₈, R₉ and R₄₀ are as defined hereinbefore,
which may be reduced to yield a compound of formula (I/d) : 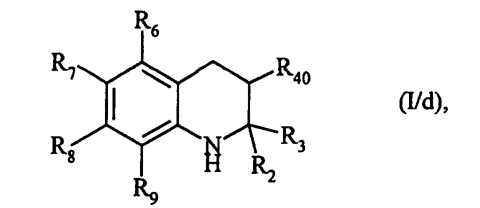 a particular case of the compounds of formula (I) wherein R₂, R₃, R₄₀, R₆, R₇, R₈ and R₉ are as defined hereinbefore,
the compounds (I/a) to (I/d) constituting the totality of the compounds of formula (I) :
- which may be, if necessary, purified according to a conventional purification technique,
- the stereoisomers of which are, where appropriate, separated according to a conventional separation technique,
- which are, if desired, converted into their addition salts with a pharmaceutically acceptable acid or base.

8. Pharmaceutical compositions comprising as active ingredient at least one compound according to any one of claims 1 to 6, alone or in combination with one or more pharmaceutically acceptable, inert, non-toxic excipients or carriers.

9. Pharmaceutical compositions according to claim 8 comprising at least one active ingredient according to any one of claims 1 to 6 for use as anti-oxidation agents in the treatment of disorders associated with ageing, such as atherosclerosis and cataract, in the treatment of cognitive disorders, in the treatment of acute neurodegenerative disorders, such as cerebral ischaemia and epilepsy, and in the treatment of chronic neurodegenerative disorders, such as Alzheimer's disease, Pick's disease and neurodegeneracies of the basal ganglia (Parkinson's disease, Huntington's disease).
